# EUROPEAN PATENT SPECIFICATION

(11) **EP 0 586 605 B1**
(45) Date of publication and mention of the grant of the patent: **05.08.1998**
(21) Application number: 92914861.7
(22) Date of filing: 10.04.1992
(51) Int. Cl.: C12Q 1/00, C12Q 1/68, G01N 33/53, G01N 33/536, G01N 33/537, G01N 33/538, G01N 33/541, G01N 33/543, G01N 33/544, G01N 33/546, G01N 33/532, G01N 33/545

(54) **REAGENTS AND METHODS FOR PERFORMING TWO-STEP ION-CAPTURE BINDING ASSAYS**
REAGENZIEN UND VERFAHREN ZUR AUSFÜHRUNG EINES ZWEISTUFIGEN IONENEINFANG-ASSAY
REACTIFS ET PROCEDES PERMETTANT DE REALISER DES ANALYSES DE FIXATION AVEC CAPTURE D'IONS EN DEUX ETAPES

(30) Priority: 30.05.1991 US 707478
(43) Date of publication of application: 16.03.1994
(73) Proprietor: ABBOTT LABORATORIES, Abbott Park, Illinois 60064-3500 (US)
(72) Inventor: ADAMCZYK, Janina, Gurnee, IL 60031 (US); BERRY, Daniel, S., Libertyville, IL 60048 (US); FICO, Rosario, Zion, IL 60099 (US); JOU, Yi-Her, Libertyville, IL 60048 (US); STROUPE, Stephen, D., Libertyville, IL 60048 (US)
(74) Representative: Modiano, Guido, Dr.-Ing.
(86) International application number: US9202980
(87) International publication number: WO9221770

(56) References cited:
- EP-A- 0 326 100
- EP-A- 0 406 473
- US-A- 4 121 975
- US-A- 4 530 900
- US-A- 4 780 409
- US-A- 4 935 147
- US-A- 5 051 356
- US-A- 5 094 962
- ANNALES DE BIOLOGIE CLINIQUE, vol. 48, no. 7, 1990 PARIS F, pages 467-471, J.F. DELAGNEAU ET AL. 'Criteria for the selection of a solid phase to be used in immunoassays.'

## Description

### BACKGROUND OF THE INVENTION

### 1. Field of the Invention

This invention relates generally to the field of binding assay devices and methods.

### 2. Description of Related Art

Various analytical procedures and devices are commonly employed in assays to determine the presence and/or concentration of substances of interest or clinical significance which may be present in biological liquids or other materials. Such substances are commonly termed "analytes" and can include antibodies, antigens, drugs, hormones, etc.

Immunoassay techniques take advantage of the mechanisms of the immune systems of higher organisms, wherein antibodies are produced in response to the presence of antigens which are pathogenic or foreign to the organisms. These antibodies and antigens, i.e., immunoreactants, are capable of binding with one another, thereby creating a highly specific reaction mechanism which can be used in vitro to determine the presence or concentration of that particular antigen in a biological sample.

There are several known immunoassay methods using immunoreactants, wherein at least one of the immunoreactants is labeled with a detectable component so as to be analytically identifiable. For example, the "sandwich" or "two-site" technique may involve the formation of a ternary complex between an antigen and two antibodies. A convenient method of detecting complex formation in such a technique is to provide one labeled antibody and an unlabeled antibody bound to a solid phase support such that the complex can readily be isolated. In this example, the amount of labeled antibody associated with the solid phase is directly proportional to the amount of analyte in the test sample.

An alternative technique is the "competitive" assay. In one example of a competitive assay, the capture mechanism again may use an antibody attached to an insoluble solid phase, but a labeled analyte (rather than a labeled antibody) competes with the analyte present in the test sample for binding to the immobilized antibody. Similarly, an immobilized analyte can compete with the analyte of interest for a labeled antibody. In these competitive assays, the quantity of captured labeled reagent is inversely proportional to the amount of analyte present in the sample.

Despite their great utility, there are disadvantages with such assay methods. First, the heterogenous reaction mixture of liquid test sample and soluble and insoluble assay reagents, can retard the kinetics of the reaction. In comparison to a liquid phase reaction wherein all reagents are soluble, i.e. a homogeneous reaction mixture, the heterogenous reaction mixture can require longer incubation periods for equilibrium to be reached in the reaction mixture between the insoluble solid phase system, the free analyte in the test sample, the soluble labeled reagent, and the newly formed insoluble complex. Second, conventional methods of attaching binding members to the solid phase, such as adsorption of antibody to the solid phase, can produce a solid phase which will readily bind substances other than the analyte. This is referred to as nonspecific binding and can interfere with the detection of a positive result. Third, with conventional immobilization methods, separate batches of manufactured solid phase reagents can contain variable amounts of immobilized binding member.

With regard to the manufacture of solid phase devices for use in binding assays, there are a number of assay devices and procedures wherein the presence of an analyte is indicated by the analyte's binding to a labeled reagent and/or a complementary binding member that is immobilized on a solid phase such as a dipstick, teststrip, flow-through pad, paper, fiber matrix or other solid phase material. Such a specific binding reaction results in a distribution of the labeled reagent between that which is immobilized upon the solid phase and that which remains free. Typically, the presence or amount of analyte in a test sample is indicated by the extent to which the labeled reagent becomes immobilized upon the solid phase.

The use of porous teststrips in the performance of specific binding assays is also well-known. In a sandwich assay procedure, a test sample is applied to one portion of the teststrip and is allowed to migrate through the strip material by means of capillary action. The analyte to be detected or measured passes through the teststrip material, either as a component of the fluid test sample or with the aid of an eluting or chromatographic solvent which can be separately added to the strip. The analyte is thereby transported into a detection zone on the teststrip wherein an analyte-specific binding member is immobilized. The extent to which the analyte becomes bound in the detection zone can be determined with the aid of a labeled analyte-specific binding member which may be incorporated in the teststrip or which may be applied separately to the strip.

Examples of devices based upon these principles include those described the following patents and patent applications. Deutsch et al. describe a quantitative chromatographic teststrip device in U.S. Pat. Nos. 4,094,647, 4,235,601 and 4,361,537. The device comprises a material capable of transporting a solution by capillary action. Different areas or zones in the strip contain the reagents needed to perform a binding assay and to produce a detectable signal as the analyte is transported to or through such zones. The device is suited for chemical assays as well as binding assays which are typified by the binding reaction between an antigen and a complementary antibody.

Many variations on the device of Deutsch et al. have been subsequently disclosed. For example, Tom et al. (U.S. Pat. No. 4,366,241) disclose a bibulous support with an immunosorbing zone, containing an immobilized specific binding member. The test sample is applied to the immunosorbing zone, and the assay result is read at the immunosorbing zone.

Weng et al. (United States Patent Nos. 4,740,468 and 4,879,215) also describe a teststrip device and methods for performing a binding assay. The device is used with a test solution containing the test sample, suspected of containing the analyte of interest, and a labeled specific binding member which binds to the analyte. The assays involve both an immobilized second binding member, which binds to the labeled binding member, and an immobilized analog of the analyte, which removes unbound labeled binding member from the assay system. Greenquist et al. (United States Patent Nos. 4,806,311 and 4,806,312) describe a layered assay device for performing binding assays similar to those of Weng et al., wherein a first immobilized reagent such as an analyte-analog is used to remove unbound materials from the reaction mixture prior to the passage of the reaction mixture passage to a subsequent detection layer.

Rosenstein (EP-A-0 284 232) and Campbell et al. (United States Patent No. 4,703,017) describe assay methods and devices for performing specific binding assays, wherein the preferred detectable label is a colored particle consisting of a liposome containing a dye. Bahar, et al. (United States Patent No. 4,868,108) describe an assay method and device for performing a specific binding assay, wherein the device involves a multizoned support through which test sample is transported and an enzyme/substrate detection means. Eisinger et al. (United States Patent No. 4,943,522) describe an assay method and a device for performing specific binding assays, using a multizoned large-pored lateral flow membrane through which test sample is transported by capillary action.

Ullman et al. (EP-A-0 271 204) is related to the previously described Weng et al. patents (United States Patent Nos. 4,740,468 and 4,879,215). Ullman et al. describe the preparation of a test solution containing an analyte-analog and a test sample suspected of containing the analyte. The test solution is contacted to a bibulous material having two sequential binding sites: the first binding site containing a specific binding pair member capable of binding the analyte and the analyte-analog, the second binding site capable of binding that analyte-analog which is not bound at the first binding site.

Cerny E. (WO 86/03839) describes a binding assay wherein a test solution, containing the test sample and a labeled test substance, is allowed to diffuse through a solid phase to provide a measurable diffusion pattern. The resultant diffusion pattern has a diameter which is greater than the diameter of the diffusion pattern of the labeled test substance alone.

Zuk et al. (United States Patent No. 4,956,275) describe a method and device for detecting an analyte by means of a sensor apparatus. An analyte-related signal is measured at two or more sites on the assay device by means of the sensor apparatus, and the mathematical relationship between the measurements provides a value (e.g., difference, ratio, slope, etc.) which is compared against a standard containing a known amount of analyte.

Hochstrasser (U.S. Pat. 4,059,407) discloses a dipstick device which can be immersed in a biological fluid for a semi-quantitative measurement of the analyte in the fluid. The semi-quantitative measurement of the analyte is accomplished by using a series of reagent-containing pads, wherein each pad in the series will produce a detectable color (i.e., a positive result) in the presence of an increasing amount of analyte. Also of interest in the area of dipstick devices are U.S. Pat. Nos. 3,802,842, 3,915,639 and 4,689,309.

Grubb et al. (U.S. Pat. No. 4,168,146) describe the use of a porous teststrip material to which an antigen-specific antibody is immobilized by covalent binding to the strip. The teststrip is immersed in a solution suspected of containing an antigen, and capillary migration of the solution up the teststrip is allowed to occur. As the antigen moves up the teststrip it binds to the immobilized antigen-specific antibody. The presence of antigen is then determined by wetting the strip with a second antigen-specific antibody to which a fluorescent or enzyme label is covalently bound. Quantitative testing can be achieved by measuring the length of the strip that contains bound antigen. Variations on such a teststrip are disclosed in U.S. Pat. No. 4,435,504 which employs a two enzyme indicator system; U.S. Pat. No. 4,594,327 which discloses the addition of a binding agent to whole blood samples which causes the red blood cells to aggregate at the area of the strip adjacent to the air/liquid interface; and U.S. Pat. No. 4,757,004 which discloses a means for controlling the shape of the fluid front migrating along the teststrip. The assay principle is further described in Zuk et al., Enzyme Immunochromatography - A Quantitative Immunoassay Requiring No Instrumentation, Clinical Chemistry, 31(7): 1144-1150, 1985.

Further examples of strip-type diagnostic devices include the following. Swanson et al. (EP 088 636) describe an apparatus for the quantitative determination of an analyte involving a fluid-permeable solid medium containing a predetermined number of successive spaced reaction zones. The reaction zones include a reactant capable of reacting with the analyte to produce a detectable signal; the greater the number of zones producing a detectable signal, the greater the amount of analyte in the test sample. Freisen et al. (U.S. Patent Number 4,861,711) describe a sheet-like diagnostic device containing several functional sectors through which the sample must pass. At least one of the sectors includes an immobilized reagent having a biological affinity for the analyte or an analyte complex.

Gordon et al. (U.S. Patent Number 4,956,302) describe a teststrip device characterized by having the analyte, test sample and/or eluting solvent migrate through the device in a single direction, thereby sequentially contacting reagent-containing zones or detection zones. Gordon et al. (U.S. Patent Number 4,960,691) describe a device that includes one or more bounded pathways to direct the migration of the analyte, test sample and/or eluting solvent through the reagent-containing zones and detection zones in a predetermined order.

A variety of binding methods have been used to remove an analyte from a test solution. Bolz et al. (United States Patent No. 4,020,151) describe a solid-phase assay for the quantitation of antigens or antibodies in a test sample. The sample antigen or antibody is adsorbed directly onto a solid support surface, such as anion exchange resin, and the support is then exposed to a labeled specific binding member that is immunologically reactive with the sample antigen or antibody.

Schick et al. (United States Patent No. 4,145,406) describe the use of an ion exchange adsorbent to non-specifically bind protein. Marshall et al. (United States Patent No. 4,211,763) describe a method for determining thyroid function involving an anion exchange resin to bind protein and form an agglomerate. Tabb et al. (United States Patent No. 4,362,697) describe a test device involving the use of a copolymer of vinyl pyrrolidone as an enhancer substance. Giegel et al. (United States Patent No. 4,517,288) describe a method for conducting a ligand assay requiring the adsorption or immunological binding of an analyte-specific binding member to a porous medium, followed by the application of the analyte to the porous medium.

Other assay methods involve the use of auxiliary specific binding members. Tanswell et al. (United States Patent No. 4,642,930) describe a process for determining the presence of a polyvalent antigen by incubating the antigen with three receptors; a first and a third receptor which bind to the antigen and a second receptor, bound to a solid support, which specifically binds to the first receptor. Valkirs et al. (United States Patent No. 4,727,019) describe a method and device for ligand-receptor assays, as in Tanswell et al., wherein an anti-receptor (e.g., avidin) is immobilized on a porous member and binds to a receptor (e.g., an analyte-specific antibody bound to biotin) which is bound to the target ligand. Wolters et al. (U.S. Patent Number 4,343,896) describe the use of ancillary specific binding members to prepare or complete detectable complexes, i.e., the use of a third antibody in a binding assay to complete a detectable analyte-binding member complex. W. Georghegan (United States Patent No. 4,880,751) describes a method for preparing an immunoadsorption matrix by adsorbing the F(c) portion of a selected IgG molecule onto a charged surface. Parikh et al. (U.S. Patent Number 4,298,685) describe the use of a conjugate of biotin and an anti-analyte antibody together with an inert support bearing immobilized avidin. The specific binding of the avidin and biotin components enables the immobilization of the antibody on the inert support.

Alternative separation methods include the use of a magnetic solid phase, polymerization techniques and the formation of analyte complexes having characteristics different than the non-complexed analyte. Ullman et al. (United States Patent No. 4,935,147) describe a method for separating charged suspended non-magnetic particles from a liquid medium by contacting the particles with charged magnetic particles and a chemical reagent. The chemical reagent forms non-specific bonds between the magnetic and non-magnetic particles to produce a magnetic coaggregate. A magnetic field gradient is applied to the reaction container to concentrate the coaggregate to one part of the container, and the liquid medium is then decanted.

Longoria et al. (United States Patent No. 4,948,726) describe an assay method involving the reaction of antigen and antibody molecules to form an antigen/antibody complex that uniquely exhibits an ionic charge that is different from the ionic charges of the individual molecules. A filter paper matrix is then chosen for its unique affinity for the antigen/antibody complex. Milburn et al. (United States Patent No. 4,959,303) describe an assay wherein antigen from a test sample and an antibody specific for the antigen are incubated under conditions sufficient for the antibody to bind to the support when the antigen is bound to the antibody.

Vandekerckhove (U.S. Patent No. 4,839,231) describes a two-stage, protein immobilization process involving an initial separation or isolation of target proteins in a gel, such as a polyacrylamide electrophoresis gel, followed by the transfer of those isolated proteins to the surface of a coated support for immobilization. The coated support is prepared by contacting a chemically inert support material (which material bears negatively charged groups) with a solution of either polyvinylpyridine or polybrene (which polymer bears positively charged groups). The capacity of the positively charged polymer to form ionic linkages with the negatively charged groups of the support material results in the formation of an insoluble polymeric film on the support.

Monji et al. (U.S. Patent No. 4,780,409) describe a reactant conjugated to a temperature-sensitive or salt-sensitive polymer which will precipitate from a test solution when the temperature or salt concentration of that solution is adjusted to an appropriate level. Marshall (U.S. Patent No. 4,530,900) describes a reactant conjugated to a soluble polymer, wherein the polymer is rendered insoluble for removal from solution and is physically removed from the test solution by filtration or centrifugation. Marshall discloses two means by which this reactant-polymer conjugate is rendered insoluble: the lowering of the pH of the solution or the addition of a salt as in Monji et al. Marshall goes on to describe that the insolubilized conjugate is then precipitated, removed from the test solution and finally resolubilized to form a second solution prior to the detection of analyte.

Hiltibran et al. (EP-A-0 406 473 and EP-A-0 326 100) disclose examples of ion-capture assays wherein the fibrous solid phase was positively charged by means of coating it with the polymeric quaternary compound Celquat™ L-200, which is a water soluble cellulose derivative. Other polycations for such use are also disclosed.

### SUMMARY OF THE INVENTION

The present invention provides a novel two-step binding assay method for determining the presence or amount of an analyte in a test sample. The assay involves a capture reagent, containing a first binding member conjugated to a polymeric anion substance, an indicator reagent, containing a second binding member with a detectable label and a solid phase material containing a reaction site made of a polymeric cation substance having a nitrogen content of at least about ten percent.

Alternatively, the polymeric cation substance has a nitrogen content of at least about two percent and the method uses a non-specific binding blocker comprising an unbound polymeric anion material, wherein the non-specific binding blocker inhibits the non-specific binding of reagents to the solid phase.

The specific binding members of the capture reagent and indicator are chosen for the formation of a complex with the analyte in a sandwich assay, a competitive assay or an indirect assay, thereby forming a detectable complex in proportion to the presence or amount of the analyte in the test sample.

The solid phase is contacted with the capture reagent and the test sample, whereby the polymeric cation of the solid phase attracts and attaches to the polymeric anion of the capture reagent, thereby immobilizing the capture reagent and complexes thereof upon the solid phase. The solid phase is then contacted with the indicator reagent, whereby the indicator reagent becomes bound to the immobilized capture reagent or complex thereof in proportion to the amount of analyte present in the test sample. Typically, the indicator reagent associated with the solid phase is then detected to determine the presence or amount of the analyte in the test sample. Alternatively, that indicator reagent which remains unbound can be detected.

The two-step assays of the present invention are advantageously performed when the polymeric cation substance is a polymeric quaternary ammonium compound having a nitrogen content of at least about ten percent, excluding the counter ions. The use of such a polycationic substance provides a solid phase that can be subjected to one or more washing steps without losing the capability to attract and retain the capture reagent.

In alternative embodiments, the assay protocol may further include the addition of a signal producing reagent to the indicator reagent to produce a detectable signal indicating the presence or amount of the analyte in the test sample. In yet another embodiment, the assay protocol may include the addition of an ancillary specific binding member reagent. In an indirect assay protocol, the ancillary specific binding member is capable of binding the analyte and is capable of binding to either the indicator reagent or to the capture reagent.

The specific binding member component of the capture reagent can be either a hapten or a macromolecule. The charged capture reagent enables homogeneous assay reactions wherein the reaction complexes can be removed from the reaction mixture by contacting the mixture with an oppositely charged solid phase. Virtually any binding assay (sandwich assays, competitive assays, indirect assays, assays using ancillary specific binding members, inhibition assays, etc.) can be adapted to use the ion-capture techniques of the present invention.

In the assay methods according to the present invention a) the use of liquid phase kinetics facilitates the formation of a complex from the homogeneous mixture of analyte and assay reagent specific binding members, and b) the ion-capture technique increases the potential number of complexes that can be immobilized on a solid support. If the advantages of liquid phase kinetics are not sought, the present invention also provides an efficient method of immobilizing binding members on a solid phase through a method other than absorption, adsorption or covalent binding.

### DETAILED DESCRIPTION OF THE INVENTION

The assay methods of the present invention can be used in a variety of immunoassay formats. The present invention, however, is not limited to immunoreactive assays. Any assays using specific binding reactions between the analyte and assay reagents can be performed.

### Definitions

The following definitions are applicable to the present invention.

The term "specific binding member", as used herein, refers to a member of a specific binding pair, i.e., two different molecules where one of the molecules through chemical or physical means specifically binds to the second molecule. The complementary members of a specific binding pair may also be referred to as a ligand and a receptor. In addition to the well-known example of the antigen and antibody specific binding pair, alternative specific binding pairs are exemplified by the following: biotin and avidin, carbohydrates and lectins, complementary nucleotide sequences (including probe and capture nucleic acid sequences used in DNA hybridization assays to detect a target nucleic acid sequence), complementary peptide sequences (including those formed by recombinant methods), effector and receptor molecules, hormone and hormone binding protein, enzyme cofactors and enzymes, enzyme inhibitors and enzymes, and the like. Furthermore, specific binding pairs can include members that are analogs of the original specific binding member. For example, a derivative or fragment of the anaiyte (an analyte-analog) can be used so long as it has at least one epitope in common with the analyte. Immunoreactive specific binding members include antigens, haptens, antibodies, and complexes thereof including those formed by recombinant DNA methods or peptide synthesis. An antibody can be a monoclonal or polyclonal antibody, a chimeric antibody, a recombinant protein or a mixture(s) or fragment(s) thereof, as well as a mixture of an antibody and other specific binding members. The details of the preparation of such antibodies and their suitability for use as specific binding members are well-known to those skilled-in-the-art.

The term "hapten", as used herein, refers to a partial antigen or non-protein binding member which is capable of binding to an antibody, but which is not capable of eliciting antibody formation unless coupled to a carrier protein.

The term "test sample", as used herein, refers to virtually any liquid sample. The test sample can be derived from any desired source, such as a physiological fluid, for example, blood, saliva, ocular lens fluid, cerebral spinal fluid, sweat, urine, milk, ascites fluid, mucous, synovial fluid, peritoneal fluid or amniotic fluid. The liquid test sample can be pretreated prior to use, such as preparing plasma from blood and diluting viscous liquids. Methods of pretreatment can also involve separation, filtration, distillation, concentration, inactivation of interfering components, and the addition of reagents. Besides physiological fluids, other liquid samples such as water, food products and the like can be used. In addition, a solid test sample can be used once it is modified to form a liquid medium.

The term "analyte", as used herein, refers to the substance to be detected in or separated from the test sample by means of the present invention. The analyte can be any substance for which there exists a naturally occurring specific binding member or for which a specific binding member can be prepared. In addition, the analyte may bind to more than one specific binding member. "Analyte" also includes any antigenic substances, haptens, antibodies, and combinations thereof. The analyte can include, but is not limited to, a protein, a peptide, an amino acid, a hormone, a steroid, a vitamin, a drug including those administered for therapeutic purposes as well as those administered for illicit purposes, a bacterium, a virus, and metabolites of or antibodies to any of the above substances.

The term "analyte-analog", as used herein, refers to a substance which cross-reacts with a binding member specific for the analyte, although the analyte-analog may react with the binding member to a greater or a lesser extent than does the analyte itself. The analyte-analog can include a modified analyte as well as a fragmented or synthetic portion of the analyte molecule so long as the analyte-analog has at least one epitopic site in common with the analyte of interest.

The term "label", as used herein, refers to any substance which directly or indirectly attaches to a specific binding member and which is capable of producing a signal that is detectable by visual or instrumental means. Various suitable labels for use in the present invention can include chromogens; catalysts; fluorescent compounds; chemiluminescent compounds; radioactive labels; direct visual labels including colloidal metallic and non-metallic particles, dye particles, enzymes or substrates, or organic polymer latex particles; liposomes or other vesicles containing signal producing substances.

A large number of enzymes suitable for use as labels are disclosed in U.S. Patent No. 4,275,149, columns 19-23.

For example, an enzyme/substrate signal producing system useful in the present invention involves the enzyme alkaline phosphatase and the substrate nitro blue tetrazolium-5-bromo-4-chloro-3-indolyl phosphate or a derivative or analog thereof.

In an alternative signal producing system, the label can be a fluorescent compound where no enzymatic manipulation of the label is required to produce a detectable signal. Fluorescent molecules such as fluorescein, phycobiliprotein, rhodamine and their derivatives and analogs are suitable for use as labels in this system.

In an especially preferred embodiment of the present invention, a visually detectable, colored particle can be used as the label component of the indicator reagent, thereby providing for a direct colored readout of the presence or concentration of the analyte in the sample without the need for the addition of other signal producing reagents. Materials for use as the colored particles are colloidal metals, such as gold, and dye particles as disclosed in U.S. Pat. Nos. 4,313,734 and 4,373,932.

The preparation and use of non-metallic colloids, such as colloidal selenium particles, are disclosed in U.S. Patent No. 4,954,452.

The use of colloidal particle labels in immunochromatography is disclosed in EP-A-0 299 428.

Organic polymer latex particles for use as labels are disclosed in EP-A-0 360 088.

The term "signal producing component", as used herein, refers to any substance capable of reacting with the analyte or another assay reagent to produce a reaction product or signal that indicates the presence or amount of the analyte and that is detectable by visual or instrumental means. "Signal production system", as used herein, refers to the group of assay reagents that are used to produce the desired reaction product or signal. For example, one or more signal producing components can be used to react with a label and generate the detectable signal. For example, when the label is an enzyme, amplification of the detectable signal is obtained by reacting the enzyme with one or more substrates or additional enzymes to produce a detectable reaction product.

The term "indicator reagent", as used herein, refers to a specific binding member which is attached or which becomes attached to a label. The indicator reagent produces a detectable signal at a level relative to the amount of an analyte in the test sample. Generally, the indicator reagent is detected or measured after it is captured on the solid phase material, but the unbound indicator reagent can also be measured to determine the result of an assay.

The specific binding member of the indicator reagent is capable of binding either to the analyte as in a sandwich assay, to the capture reagent as in a competitive assay, or to an ancillary specific binding member to complete a detectable complex. The label, as described above, enables the indicator reagent to produce a detectable signal that is related to the presence or amount of analyte in the test sample. The specific binding member component of the indicator reagent enables the indirect binding of the label to the analyte, to an ancillary specific binding member or to the capture reagent. The selection of a particular label is not critical, but the label will be capable of generating a detectable signal either by itself, such as a visually detectable signal generated by colored organic polymer latex particles, or in conjunction with one or more additional signal producing components, such as an enzyme/substrate signal producing system. A variety of different indicator reagents can be formed by varying either the label or the specific binding member; it will be appreciated by one skilled-in-the-art that the choice involves consideration of the analyte to be detected and the desired means of detection.

As mentioned above, the label can become attached to the specific binding member during the course of the assay. For example, a biotinylated anti-analyte antibody may be reacted with a labeled streptavidin molecule. Any suitable combination of binding members and labels can be used.

The term "capture reagent", as used herein, refers to an unlabeled specific binding member which is attached to a charged substance. The attachment of the components is essentially irreversible and can include covalent mechanisms. The specific binding member can be a small molecule, such as a hapten or small peptide, so long as the attachment to the charged substance does not interfere with the binding member's binding site. The binding member component of the capture reagent is specific either for the analyte as in a sandwich assay, for the indicator reagent or analyte as in a competitive assay, or for an ancillary specific binding member, which itself is specific for the analyte.

The charged substance component of the capture reagent includes anionic polymers. For example, anionic polymers include polyglutamic acid (PGA), anionic protein or derivitized protein such as albumin, anionic polysaccharides such as heparin or alginic acid, polyaspartic acid, polyacrylic acid, and polyamino acids having a net negative charge at a pH appropriate for the specific binding reaction (such as a pH in the range of 4 to 10.) Furthermore, the specific binding member can be joined to more than one charged polymer to increase the net charge associated with the capture reagent.

The capture reagents disclosed herein are used to facilitate the observation of the detectable signal by substantially separating the analyte and/or the indicator reagent from other assay reagents and the remaining test sample components. In its most advantageous use, the capture reagent is reacted with the test sample and assay reagents in a homogeneous reaction mixture. Following the formation of the desired specific binding member complexes, the complexes involving a capture reagent are removed from the homogeneous reaction mixture by contacting the homogeneous reaction mixture to a solid phase that is oppositely charged with respect to the charge of the capture reagent.

A negatively charged capture reagent can be prepared by conjugating the selected specific binding member to one or more activated polymeric anionic molecules and conjugate bases thereof represented by the general formula: 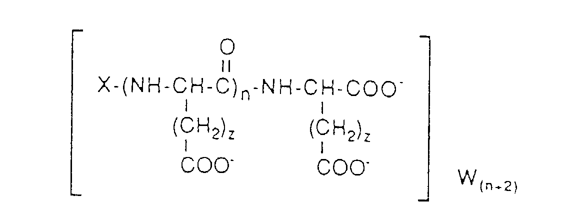 wherein n is about 10 to about 500; z is about 1 to about 6; W is chosen from H⁺, Na⁺, K⁺, Li⁺, amine salts such as H⁺NR₃, and derivatives thereof; and X is virtually any reactive group or moiety having a reactive group that enables the chemical binding of the specific binding member and the polymer. "X" can be an amine-reactive group or moiety, a thiol-reactive group or moiety, or a thiol group or moiety represented by -A-SH wherein A is a spacer arm. For example, a specific binding member having an amino group can be conjugated to an activated PGA anionic molecule having an amine-reactive moiety. The amine-reactive moieties enable the binding of the activated polymer to an amino group on a specific binding member and include, but are not limited to, those represented by the following formulas: 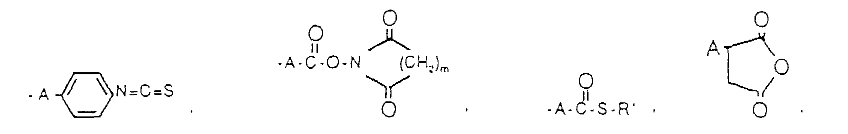 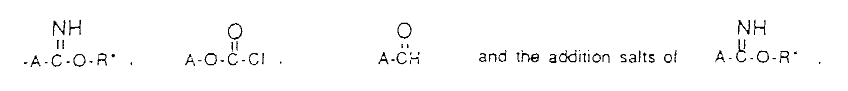 wherein m is two or three, R' is a sulfur stabilizer and R" is an aliphatic or aryl group. Sulfur stabilizers include, but are not limited to, 2-pyridyl, 4-pyridyl and 5-nitro-2-pyridyl groups. "A" represents a spacer of about one to about thirty atoms including, but not limited to, carbon, nitrogen, sulfur and oxygen atom chains and combinations thereof such as polyether, polymethylene and polyamide, as well as aromatic spacers such as phenylthiocarbamyl.

Alternatively, a specific binding member having a thiol group can be conjugated to an activated polymer having a thiol-reactive moiety. The thiol-reactive moieties include, but are not limited to, those represented by the following formulas: 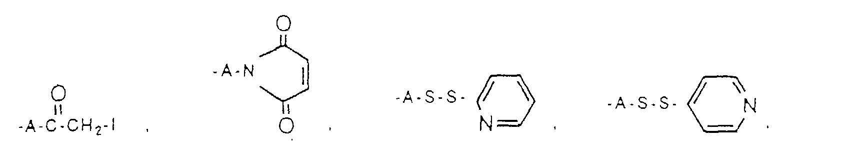 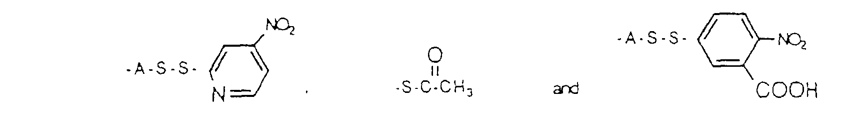 wherein A is a spacer of about one to about thirty atoms as described above. In yet another alternative, a specific binding member having a thiol-reactive group can be linked to an activated polymer having a thiol moiety such as -A-SH.

Typically, the negatively charged capture reagents of the following Examples were formed by reacting the desired specific binding member with an activated PGA molecule having modified terminal amino groups. Briefly, the modification method involved: 1) dissolving the PGA in a solvent (e.g., a water miscible aprotic solvent such as dioxane, dimethylformamide, 1-methyl-2-pyrrolidinone and dimethyl sulfoxide); 2) adding a proton absorbing reagent (e.g., 4-methyl morpholine) in the amount of about one equivalent per titratable carboxylic acid; 3) adding about a 2 to about a 100 molar excess of an amine-reactive modification reagent (e.g., 1,4-phenylene diisothiocyanate dissolved in dimethylformamide); 4) reacting the mixture; and 5) removing the unreacted amine-reactive modification reagent. Suitable proton absorbing reagents include alkali metal hydroxides such as sodium hydroxide, potassium hydroxide or lithium hydroxide, and tertiary amines such as 4-methyl morpholine and triethylamine.

The polymeric anionic molecule or the specific binding member will include one or more amino, carboxyl or thiol groups, or can be activated by the incorporation of an amino, carboxyl or thiol group, thereby enabling the chemical cross-linking of the specific binding member with the polymeric anionic molecule. "Activated species" refer to specific binding members and polymeric anionic molecules which contain a reactive group through the incorporation of a cross-linking or other activating agent. The amine-reactive modification reagents are a subclass of those reagents used to "activate" a specific binding member or polymeric anionic molecule, i.e., to prepare the specific binding member or the polymeric anionic molecule for chemical cross-linking. Activating agents also include thiol introducing agents such as the thiolanes (such as 2-iminothiolane), succinimidyl mercaptoacetates (such as N-succinimidyl-S-acetylmercaptoacetate), and disulfide compounds which are subsequently reduced to a thiol. The thiol introducing agents can be used to activate specific binding members and solid phase materials for their subsequent reaction with a thiol-reactive group.

Amine-reactive modification reagents include, but are not limited to, bifunctional crosslinking or coupling agents, such as succinic anhydride analogs, iminothiolane analogs, homobifunctional reagents and heterobifunctional reagents. which enable the chemical cross-linking of the specific binding member and the polymeric anionic molecule. Examples of homobifunctional reagents can be represented by the formula X-A-X wherein X is an amine-reactive group and A is a spacer of about one to about thirty atoms. Examples of heterobifunctional reagents can be represented by the formula X-A-Y, wherein X is an amine-reactive group, Y is a thiol-reactive moiety, a thiol moiety or a thiol precursor and A is a spacer of about one to about thirty atoms as described above. Proteinaceous specific binding members with cysteine residues at the protein's active site can have their activity decreased by the addition of a coupling agent, therefore the cysteine residues in the active site must be protected, by means known in the art, prior to reacting the protein with the coupling agent.

The term "coupling agent", as used herein, includes bifunctional crosslinking or coupling agents, i.e., molecules containing two reactive groups or "ends", which may be tethered by a spacer. The reactive ends can be any of a variety of functionalities including, but not limited to: amino reacting ends such as N-hydroxysuccinimide (NHS) active esters, imidoesters, aldehydes, epoxides, sulfonyl halides, isocyanate, isothiocyanate, and nitroaryl halides; and thiol reacting ends such as pyridyl disulfides, maleimides, thiophthalimides, and active halogens. The heterobifunctional crosslinking reagents have two different reactive ends, e.g., an amino-reactive end and a thiol-reactive end, while homobifunctional reagents have two similar reactive ends, e.g., bismaleimidohexane (BMH) which permits the cross-linking of sulfhydryl-containing compounds, and NHS homobifunctional crosslinkers such as disuccinimidyl suberate (DSS) as well as the water soluble analogs, sulfo-NHS esters (Pierce 1989 Handbook and General Catalog; Pierce, Rockford, IL).

Other commercially available homobifunctional cross-linking reagents include, but are not limited to, the imidoesters such as dimethyl adipimidate dihydrochloride (DMA); dimethyl pimelimidate dihydrochloride (DMP); and dimethyl suberimidate dihydrochloride (DMS). The iminothiolane analogs can be represented by the general formula: 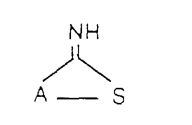 wherein A is a spacer of about 1 to about 5 atoms, e.g., 2-iminothiolane (Traut's Reagent.)

Commercially available heterobifunctional reagents suitable for use in the present invention include, but are not limited to, maleimido-NHS active esters coupling agents such as m-maleimidobenzoyl-N-hydroxy-succinimide ester (MBS); succinimidyl 4-(N-maleimidomethyl)cyclohexane-1-carboxylate (SMCC); succinimidyl 4-(p-maleimidophenyl)butyrate (SMPB) and derivatives thereof, including sulfosuccinimidyl derivatives such as sulfosuccinimidyl 4-(N-maleimido-methyl) cyclohexane-1-carboxylate (sulfo-SMCC); m-maleimidobenzoyl-sulfosuccinimide ester (sulfo-MBS) and sulfosuccinimidyl 4-(p-maleimidophenyl)butyrate (sulfo-SMPB) (Pierce). Other suitable heterobifunctional reagents include commercially available active halogen-NHS active esters coupling agents such as N-succinimidyl bromoacetate and N-succinimidyl(4-iodoacetyl)aminobenzoate (SIAB) and the sulfosuccinimidyl derivatives such as sulfosuccinimidyl(4-iodoacetyl)aminobenzoate (sulfo-SIAB) (Pierce). Another group of coupling agents is the heterobifunctional and thiol cleavable agents such as N-succinimidyl 3-(2-pyridyldithio)propionate (SPDP) (Pierce).

Yet another group of coupling agents includes the extended length heterobifunctional coupling agents described in U.S. Patent 5,002,883.

The extended length heterobifunctional coupling agents include maleimido-NHS active ester reagents wherein the spacer is represented by the formula: 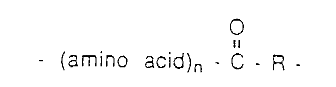 wherein the amino acid is a substituted or unsubstituted amino acid, having from three to ten carbon atoms in a straight chain; n is from one to ten; and R is an alkyl, cycloalkyl, alkyl-cycloalkyl or an aromatic carboxylic ring. The term alkyl-cycloalkyl includes alkyl groups linked to cycloalkyl ring structures where the alkyl group links the cycloalkyl to a maleimide or carbonyl group. The term alkyl includes straight or branched alkyl groups, preferably lower alkyl groups having from one to six carbon atoms.

If a spacer is present, the spacer can be any molecular chain that is nonreactive, stable and non-binding to the analyte or other specific binding members with which it will be used. The length of the spacer can be varied and can range from the size of a single atom, to the sizes disclosed in U.S. Patent 5,002,883 and EP-A-0 314 127, or larger.

The choice of the amine-reactive modification reagent, thiol introducing agent or other activating agent is not critical, but one skilled-in-the-art will know of suitable or preferred agents for use with the particular polymeric anionic molecule and specific binding member to be used in the diagnostic assay. Therefore, it will be appreciated by those skilled-in-the-art that the coupling agent or activating agent used in a given assay will generally be determined empirically.

Suitable thiol-reactive moieties of the heterobifunctional reagents include, but are not limited to, those represented by the following formulas:

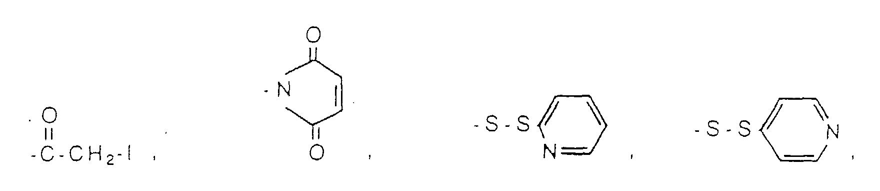

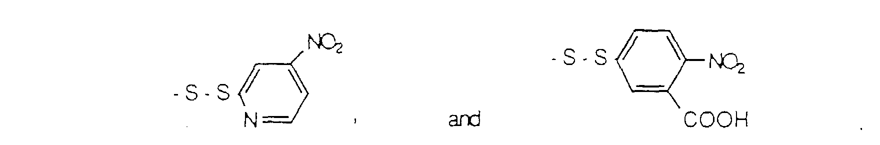

Suitable thiol precursor moieties include, but are not limited to, those represented by the following formulas:

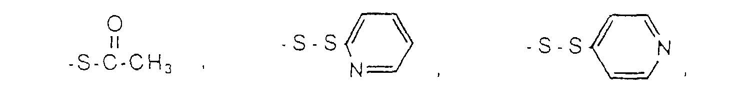

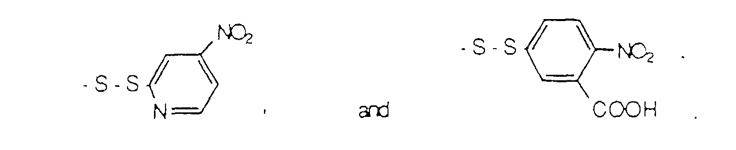

Suitable amine-reactive moieties include, but are not limited to, those represented by the following formulas: 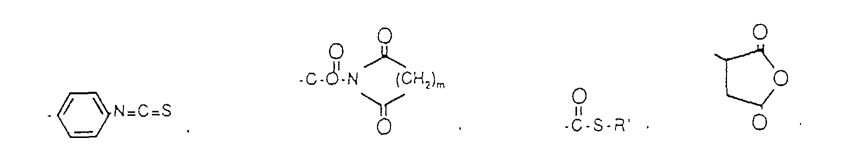 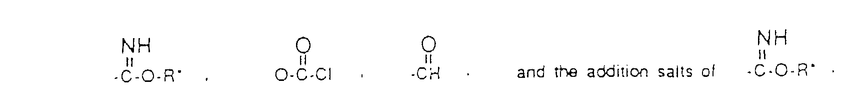 wherein m is 2 or 3, R' is a sulfur stabilizer, as described above, and R" is an aliphatic or aryl group.

In yet another method of preparing a capture reagent, a specific binding member having an amine-reactive group (e.g., an activated specific binding member) can be conjugated to a terminal amino group of the polymeric anionic molecule. Briefly, an example of a conjugation procedure involves: 1) dissolving PGA in a solvent (e.g., a water miscible aprotic solvent such as dioxane, dimethylformamide, 1-methyl-2-pyrrolidinone and dimethyl sulfoxide); 2) adding a proton absorbing reagent (e.g., an alkali metal hydroxide such as sodium hydroxide, potassium hydroxide, or lithium hydroxide, or a tertiary amine such as 4-methyl morpholine or triethylamine) in the amount of about one equivalent per titratable carboxylic acid; 3) adding about a 2 to about a 100 molar excess of amine-reactive specific binding member (e.g., phosgene-activated phenylcyclidine or phenylcyclidine-4-chloroformate); 4) reacting the mixture and 5) removing the unreacted amine-reactive specific binding member. Examples of suitable amine-reactive groups on specific binding members include, but are not limited to, the following: 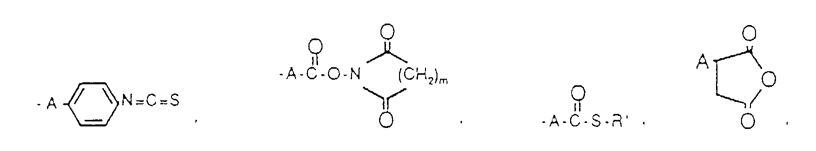 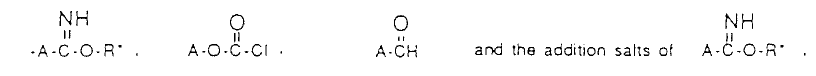 wherein A is a spacer of about one to about thirty atoms as described above, m is two or three, R' is a sulfur stabilizer and R" is an aliphatic or aryl group.

An example of the preparation of a negatively charged capture reagent involves the reaction of a specific binding member (SBM) having an amino group and an activated PGA having an amine-reactive moiety. The resulting reaction and reaction product can be illustrated as follows:

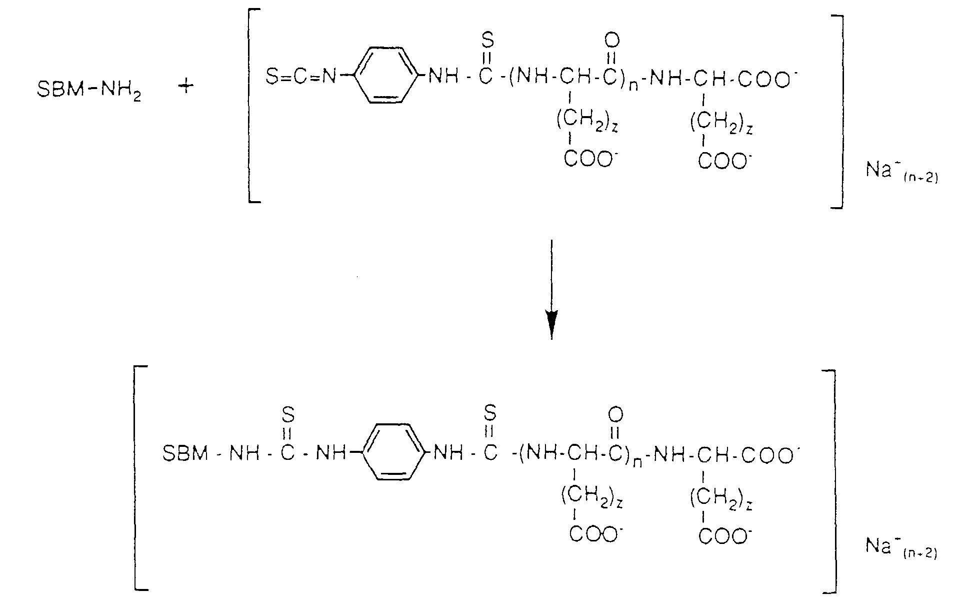

In yet another embodiment of the assay methods of the present invention, a preferred anionic polymer for use in the capture reagent is carboxymethylamylose (CMA) due to its particular performance in various immunoassay configurations. The improved performance of capture reagents containing CMA can be attributed to the higher avidity of the CMA capture reagent for the cationic solid phase. This attribute is particularly advantageous in a two step sandwich assay format wherein a polyanion is used to block nonspecific binding of the indicator reagent to the cationic solid phase.

The term "ancillary specific binding member", as used herein, refers to any member of a specific binding pair which is used in the assay in addition to the specific binding members of the capture reagent and the indicator reagent. For example, in an assay an ancillary specific binding member may bind the analyte to a second specific binding member to which the analyte itself could not attach, or as in an inhibition assay the ancillary specific binding member may be a reference binding member. One or more ancillary specific binding members can be used in an assay.

The term "solid phase", as used herein, refers to any material which is insoluble, or can be made insoluble by a subsequent reaction and can be pretreated with and retain a charged substance that is oppositely charged with respect to the charged substance of the capture reagent. In the present invention, an anionic substance is bound to a specific binding member to form the capture reagent, and a cationic substance is applied to and retained by the solid phase. Natural, synthetic or naturally occurring materials that are synthetically modified can be used as the cationic substance in the method according to Claim 1, provided they have a nitrogen content of at least about 10% (exclusive of counter ion), such as Merquat-100® compound (a cationic homopolymer of dimethyldiallylammonium chloride; Calgon Corporation, Pittsburgh, PA). Merquat-100® compound has a nitrogen content of about 10% (exclusive of counter ion). The method of Claim 1 is based on the unexpected discovery that the cationic homopolymer of dimethyldiallylammonium chloride and other polycationic substances having a nitrogen content above about 10 % (exclusive of counter ion) are advantageous in preparing a solid phase that will undergo washing during the assay process. The use of such a polycationic substance to prepare a suitably charged solid phase resulted in a solid phase that could be subjected to a greater degree of manipolation without losing the capability to attract and retain the oppositely charged capture reagent.

In the method according to Claim 6, a non-specific binding blocker comprising an unbound polymeric anion material which inhibits the non-specific binding of reagents to the solid phase is employed. In such method, natural, synthetic, or naturally occurring materials that are synthetically modified can be used as the cationic substance, provided they have a nitrogen content of at least about 2% (exclusive of counter ion), although a nitrogen content of at least about 5% (exclusive of counter ion) is preferred and a nitrogen content of at least about 10% (exclusive of counter ion) is most preferred. A wide variety of proprietary polycations are available including tertiary and quaternary ammonium compounds and derivatives of ammonium compounds. Such polycationic materials having a nitrogen content of at least about 2% include, but are not limited to, hexadimethrine bromide (Polybrene®; Sigma Chemical Company, St. Louis, Mo), GAFQuat™ quaternary ammonium compounds (GAF Corporation, Wayne, NJ, 07470), diethylaminoethyl-dextran (Sigma), Merquat-100® compound and water soluble cellulose derivatives such as diallyldimethylammonium chloride-hydroxyethyl cellulose polymer (Celquat® L-200 polymeric quaternary ammonium compounds, National Starch & Chemical Corporation, Bridgewater, NJ, 08807).

An assay device based on the ion-capture technique can have many configurations, several of which are dependent upon the material chosen as the solid phase. In various device embodiments, the solid phase may involve polymeric or glass beads, microparticles, magnetic particles, tubes, sheets, plates, slides, wells, tapes, test tubes, layered films or the like, or any other material which can retain a charged substance.

The ion-capture devices used in assay methods according to the present invention may involve a solid phase made of any suitable porous material. By "porous" is meant that the material is one through which the test sample can easily pass by capillary or wicking action and includes both bibulous and non-bibulous solid phase materials. For example, the solid phase can include a fiberglass, cellulose, or nylon pad for use in a flow-through assay device having one or more layers containing one or more of the assay reagents; a dipstick for a dip and read assay; a teststrip for wicking or capillary action (e.g., paper, nitrocellulose, polyethylene) techniques; or other porous or open pore materials well-known to those skilled-in-the-art (e.g., polyethylene sheet material as manufactured by Porex Technologies Corporation, Fairburn, Georgia, USA).

Natural, synthetic, or naturally occurring materials that are synthetically modified, can be used as a solid phase including polysaccharides, e.g., cellulose materials such as paper and cellulose derivatives such as cellulose acetate and nitrocellulose; silica; inorganic materials such as deactivated alumina, diatomaceous earth, MgSO₄, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon); porous gels such as silica gel, agarose, dextran, and gelatin; polymeric films such as polyacrilamide. The solid phase should have reasonable strength or strength can be provided by means of a support, and it should not interfere with the production of a detectable signal.

Preferred solid phase materials for flow-through assay devices include filter paper such as a porous fiberglass material or other fiber matrix materials as well as isotropically porous materials such as a polyethylene pad. The thickness of the material used will be a matter of choice, largely based upon the properties of the sample or analyte being assayed, e.g., the fluidity of the test sample.

To change or enhance the intrinsic charge of the solid phase, a polymeric cationic substance can be applied directly to the solid phase material or to microparticles which are then retained by a solid phase support material. Alternatively, coated microparticles alone can be used as the charged solid phase by being retained in a column. By "retained" is meant that the particles on or in the solid phase support material are not capable of substantial movement to positions elsewhere within the support material. The particles can be selected by one skilled-in-the-art from any suitable type of particulate material and include those composed of polystyrene, polymethylacrylate, polypropylene, latex, polytetrafluoroethylene, polyacrylonitrile, polycarbonate, glass or similar materials. The size of the particles is not critical, although it is preferred that the average diameter of the particles be smaller than the average pore size of the support material being used

Typically, the teststrip and flow-through devices employing the ion-capture principles disclosed herein are characterized by having the analyte, test sample and/or eluting solvent migrate through the device in a single direction, thereby sequentially contacting reagent-containing zones or detection zones. Alternatively, the devices used for carrying out the present invention may be configured such that the analyte migrates radially from the sample application site to the reagent-containing zones or detection zones. In yet another embodiment, the devices may include one or more bounded pathways to direct the migration of the analyte, test sample and/or eluting solvent through the reagent-containing zones and detection zones in a predetermined order.

### Uses for Ion-Capture Reagents

In accordance with the disclosure of the present invention, a sandwich assay can be performed wherein the capture reagent involves an analyte-specific binding member which has been bound to a polymeric anion substance.

The capture reagent is contacted with a test sample, suspected of containing the analyte, and an indicator reagent comprising a labeled analyte-specific binding member. The reagents can be mixed simultaneously or added sequentially, either singly or in combination to form a homogenous reaction mixture.

In the exemplary sandwich assay, a binding reaction results in the formation of a capture reagent/analyte/indicator reagent complex. The resultant complex is then removed from the excess assay reagents and test sample of the homogenous reaction mixture by means of a solid phase that retains an oppositely charged (polymeric cationic) substance.

In the ion-capture assays, the oppositely charged solid phase attracts and attaches to the capture reagent/analyte/indicator reagent complex through the interaction of the anionic and cationic polymers. The complex retained on the solid phase is then detected by examining the solid phase for the indicator reagent. If analyte is present in the sample, then label will be present on the solid phase. The amount of label on the solid phase is proportional to the amount of analyte in the sample. The only major limitation inherent in the sandwich assay is the requirement for the analyte to have a sufficient size and appropriately orientated epitopes to permit the binding of at least two specific binding members. Other sandwich assays may involve one or more ancillary specific binding members to bind the analyte to the indicator reagent and/or capture reagent.

The present invention also includes a competitive assay. In an exemplary competitive assay, the soluble capture reagent again includes a specific binding member which has been attached to a polymeric anion substance. The capture reagent is contacted, either sequentially or simultaneously, with the test sample and an indicator reagent that includes a second binding member which has been labeled with a signal generating compound. Either the capture reagent and analyte can compete in binding to the indicator reagent (e.g., the capture reagent and analyte are antigens competing for a labeled antibody), or the indicator reagent and analyte can compete in binding to the capture reagent (e.g., the indicator reagent is a labeled antigen which competes with the antigen analyte for binding to the antibody component of the capture reagent). A competitive binding or displacement reaction occurs in the homogeneous mixture and results in the formation of capture reagent/analyte complexes and capture reagent/indicator reagent complexes.

The resultant complexes are removed from the excess assay reagents and test sample by contacting the reaction mixture with the oppositely charged solid phase. The capture reagent complexes are retained on the solid phase through the interaction of the oppositely charged polymers. The complexes retained on the solid phase can be detected via the label of the indicator reagent. In the competitive assay, the amount of label that becomes associated with the solid phase is inversely proportional to the amount of analyte in the sample. Thus, a positive test sample will generate a negative signal. The competitive assay is advantageously used to determine the presence of small molecule analytes, such as small peptides or haptens, which have a single epitope with which to bind a specific binding partner. Other competitive assays may involve one or more ancillary specific binding members to bind the analyte to the indicator reagent and/or capture reagent.

For example, in an assay for theophylline, an anti-theophylline antibody (either monoclonal or polyclonal) can be conjugated with an anionic polymer to form a soluble capture reagent, and a competition for binding to that antibody can be established between labeled theophylline (i.e., indicator reagent) and the unlabeled theophylline of the test sample. After incubation, the homogeneous mixture can be contacted to a solid phase which retains a cationic polymer coating. The attraction between the oppositely charged ionic species of the capture reagent and the solid phase serves to separate the immunocomplex from the reaction mixture. The signal from the indicator reagent can then be detected. In this example, increased theophylline levels in the test sample will result in decreased signal generation associated with the solid phase.

In addition, the present invention includes an inhibition assay, such as the measurement of an antibody by inhibiting the detection of a reference antigen. For example, the capture reagent can include an antibody/anionic polymer conjugate and the indicator reagent can be a labeled antibody. The test sample, suspected of containing an antibody analyte, is mixed with a reference antigen with which the capture reagent and indicator reagent can form a detectable sandwich complex that can be immobilized upon the solid phase by the ion-capture reaction. The degree of inhibition of antigen uptake by the capture reagent is proportional to the amount of antibody analyte in the test sample, thus, as the concentration of the antibody analyte increases, the less reference antigen is available to complete the immobilized sandwich complex.

In general, once complex formation occurs between the analyte and the assay reagents, the oppositely charged solid phase is used as a separation mechanism: the homogeneous reaction mixture is contacted with the solid phase, and the newly formed binding complexes are retained on the solid phase through the interaction of the opposite charges of the solid phase and the capture reagent. If the user is not concerned with liquid phase kinetics. the capture reagent can be pre-immobilized on the solid phase to form a capture site.

When the complex of charged capture reagent and analyte (and/or indicator reagent) is contacted to the oppositely charged solid phase, the ionic attraction of the oppositely charged species governs the efficiency of the separation of the complex from the reaction mixture. The ionic attraction can be selected to provide a greater attraction than the immunological attraction of antibody for antigen, particularly when multiple polycationic and polyanionic species are included in the capture reagent and oppositely charged solid phase. A further advantage is that the "ion-capture" technique minimizes the nonspecific adsorption of interfering substances onto the solid phase, thereby offering improved accuracy of analysis. The ion-capture technique thereby enables the performance of an assay having a highly specific separation method, minimal nonspecific binding, and high sensitivity.

In one embodiment of the present invention, it was discovered that the addition of a nonspecific binding blocker reagent to the indicator reagent resulted in an increase in the signal to noise ratio. It was unexpectedly discovered that the nonspecific binding blocker could be a free polyanion even when the capture reagent used in the assay involved a polyanionic substance conjugated to a specific binding member. It would have been expected that the presence of a free or unbound polyanion would prevent, or at least reduce, the immobilization of the capture reagent on the solid phase. It was found, however, that the nonspecific blocker was more effective in inhibiting the direct, nonspecific binding of indicator reagent to the solid phase than it was in reducing the attachment of the polyanionic capture reagent to the polycationic solid phase. Suitable nonspecific binding blockers include, but are not limited to, dextran sulfate, heparin, carboxymethyl dextran, carboxymethyl cellulose, pentosan polysulfate, inositol hexasulfate and β-cyclodextrin sulfate.

It was also discovered that the amount of polyanionic nonspecific binding blocker added to the indicator reagent could be greater than the amount of polyanionic substance contained in the capture reagent. It was found that free polyanionic nonspecific binding blocker could be added to the indicator reagent in amounts 40,000 times the amount of polyanionic substance used in the capture reagent. Generally, the preferred amount of polyanionic blocker added to the indicator reagent is 50 to 14,000 times the amount of polyanionic substance used in the capture reagent. For two step sandwich assays, the preferred amount of polyanionic blocker added to the indicator reagent is 1000 to 2000 times that contained in the capture reagent.

An appropriate range of use can be determined for each analyte of interest. For example, in an assay to detect thyroid stimulating hormone (TSH) wherein dextran sulfate was added to the indicator reagent as a free polyanionic nonspecific binding blocker, suitable amounts of free polyanion ranged from 233 to 19,000 times that of the capture reagent, or about 0.1-8% dextran sulfate. As illustrated in the following Table, the preferred nonspecific binding blocker as well as the preferred amount of nonspecific binding blocker can be optimized for each analyte of interest.

| Nonspecific Binding Blocker in the Indicator Reagent | | |
|---|---|---|
| | % Dextran sulfate (MW 5,000) (blocker/capture reagent, w/w) | |
| Analyte | Preferred | More Preferred |
| TSH | 0.1 - 8 (233 - 19,000) | 0.5 - 2 (1,000 - 4,000) |
| T3 | 0.1 - 2 (2,000 - 40,000) | 0.1 - 0.2 (2,000 - 4,000) |

| | % Carboxymethyl cellulose (MW 250,000) (blocker/capture reagent, w/w) | |
|---|---|---|
| hOG | 0.01 - 0.25 (0.44 - 11) | 0.025 (1.1) |
| HIV | 0 - 0.2 (0 - 20,000) | 0.05 (5,000) |

Moreover, it was discovered that the polyanionic nonspecific binding blocker could be added to the assay as a separate reagent, or it could be included as free polyanion in the capture reagent, in an ancillary binding member reagent, in a buffer reagent or in some other reagent used in the assay. For example, when free polyanion is included in the capture reagent, it can enhance the signal to noise ratio by neutralizing interfering materials which are contained either in the test sample itself or in the other assay reagents, or those which were introduced during the device manufacturing process. The following Table illustrates some preferred amounts of nonspecific binding blocker for different analytes of interest, wherein the free polyanion is contained in the capture reagent itself.

| Nonspecific Binding Blocker in the Capture Reagent | | |
|---|---|---|
| | % Dextran sulfate (MW 5,000) (blocker/capture reagent, w/w) | |
| Analyte | Preferred | More Preferred |
| Digoxin | 0 - 0.004 (0 - 222) | 0..004 (222) |
| T3 | 0.004 - 0.01 (66 - 165) | 0.004 - 0.006 (66 - 99) |

Depending upon the the analyte of interest and the desired assay configuration, the preferred nonspecific binding blocker, as well as the optimization of its concentration and whether it is included as a component of another assay reagent, is selected by empirical techniques which can be performed without undue experimentation by one of ordinary skill in the art of binding assays. In only one known instance, i.e., the use of 0.005% dextran sulfate in the capture reagent of a competitive digoxin assay, was there an inhibition of the binding between the capture reagent and solid phase due to the addition of the nonspecific binding blocker.

### Ion-capture Assay Devices

As described above, ion-capture assay devices may include impermeable solid phase materials such as glass slides, magnetic particles, test tubes and plastic wells. However, it has also been discovered that the entire ion-capture assay can be performed in a porous solid phase material. The ion-capture assay devices can specifically involve any suitably absorbent, adsorbent, imbibing, bibulous, non-bibulous, isotropic or capillary possessing material (i.e., porous materials) through which a solution or fluid containing the analyte can pass. The solution can be pulled or pushed through the porous material by suction, hydraulic, pneumatic, hygroscopic, gravitational or capillary forces, or by a combination thereof.

Possible assay devices include, but are not limited to, a conventional chromatographic column, an elongated strip of porous material wherein the fluid flow is substantially linear, a sheet wherein the fluid flow is linear or radial, a pad of porous material or a device involving multiple layered sheets or pads. The devices can involve the production of teststrips as well as flow through devices. For purposes of brevity, however, the following descriptions will focus on teststrip devices, although the description of device zone can be applied to both strip-type or layered flow through-type devices. Those skilled-in-the-art will readily appreciate the applicability of the assay methods of the present invention to flow through device formats.

One advantageously used solid phase porous material for the production of a teststrip is nitrocellulose. Especially when a membranous solid phase material is used, the test sample and indicator reagent may be mixed prior to initiating fluid flow through the solid phase to obtain a controlled, reproducible binding reaction between the analyte and the indicator reagent. Alternatively, the test device can further include a premixing application pad which is in fluid flow contact with the elongated strip and which optionally contains the indicator reagent. The material of the application pad should be chosen for its ability to premix the test sample with the indicator reagent. For example, if nitrocellulose is used as the solid phase, then a hydrophilic polyethylene material or glass fiber filter paper are suitable application pad materials. Alternatively, if a solid phase material such as glass fiber filter paper is used, then the indicator reagent can be reversibly immobilized on the elongated strip itself, either at the sample application site or at another site downstream from the application site. In yet other alternative devices and methods, the indicator reagent can be added to the device as a separate reagent solution, either sequentially or simultaneously with the test sample and/or capture reagent.

In alternative embodiments, a teststrip or flow-through device may be made of a continuous piece of porous material containing diffusive or immobilized reagents to form the various reagent and detection zones. In yet another embodiment, a teststrip can be made from more than one solid phase material such that the different materials are in fluid flow contact to allow the analyte to migrate from one material to another. The different materials may contain different diffusive or immobilized assay reagents, with the individual material being assembled into an elongated strip or flow through pad device. In yet a further embodiment, two or more zones of the device may overlap. For example, the sample application zone may also contain a diffusive assay reagent (e.g., indicator reagent, capture reagent, etc.) which reacts with the analyte to form a complex or reactive product which continues to migrate to other zones in or on the device. In a further example, the sample application zone may contain an immobilized assay reagent (e.g., polymer oppositely charged with respect to the capture reagent) which immobilizes the capture reagent or capture reagent complexes for detection. Again, those skilled-in-the-art will readily appreciate the applicability of the methods of the present invention to a variety of device formats wherein the indicator reagent is immobilized by directly or indirectly binding to a capture reagent conjugate that is in turn immobilized by an oppositely charged solid phase material.

### EXAMPLES

The following Examples illustrate preferred ways of making materials and performing assay procedures using those materials. The Examples, however, are intended only to be illustrative, and are not to be construed as placing limitations upon the scope of the invention, which scope is defined solely by the claims.

### Example 1

### Activation of Poly-L-Glutamic Acid for the Formation of Anionic Capture Reagents

The following sequence of steps describes the chemistry used for the bulk preparation of protein-PGA conjugates for the formation of negatively charged capture reagents.

### a. Conversion of PGA-sodium salt to the free acid form

The sodium salt of PGA (100 mg; 7.35 x 10⁻⁶ mole; average MW 13,600; Sigma) was stirred overnight with a hydrogen form cation exchange resin (50 equivalents/glutamate residue; AG50W-X8; Bio-Rad). The resin previously had been swelled and washed in distilled water, and finally resuspended in distilled water (20 ml/7 gms dry weight of beads.) The supernatent was removed and lyophilized providing a 90% yield of the free acid form of PGA (PGAFA) as a white powder (80 mg; average MW 11,620). The free acid form was used to obtain solubility in organic solvents

### b. Preparation of ITC-PGAFA

The PGAFA was dissolved in solvent (DMF at ten milligrams/milliliter.) A proton absorbing reagent (4-methyl morpholine) was added to the solution in the amount of about one equivalent per titratable free carboxylic acid. Next, about a 100 mole excess of an amine-reactive modification reagent (1,4-phenylene diisothiocyanate [DITC] in sufficient DMF to dissolve it) was added to the solution. The reaction mixture was stirred at room temperature overnight. The reaction mixture was rotavaporated to near dryness, and methylene chloride (25 ml) was added to precipitate the ITC-PGAFA. The flocculant precipitate was centrifuged, and the methylene chloride and unreacted DITC were removed. The precipitation/centrifugation process was repeated until substantially no detectable DITC remained. The DITC was detected using thin layer chromatography on silica slides developed in methylene chloride; ITC-PGAFA remains at the origin, DITC moves with the solvent front. The remaining solid was vacuum dried to yield the ITC-PGAFA as a yellow powder.

### c. Coupling of ITC-PGAFA to protein to make capture reagents

The ITC-PGAFA (at about a 1 to about a 20 mole excess to the protein) was dissolved in 0.2 M sodium phosphate buffer at pH 8.5, with the volume held as low as possible. The pH was adjusted to 8.5 as necessary. The desired protein was added to this solution and incubated overnight at 37°C. The preparations were then fractionated using HPLC on either an analytical TSK 400 Bio-Rad column (7.5 x 300 mm, at a 1 ml/min flow rate) for 1-2 milligram protein preparations, or a TSK 4000 Beckman column (31.5 x 300 mm, at a 5 ml/min flow rate) for 2-10 milligram protein preparations. The elution buffer contained 0.1 M sodium phosphate and 0.3 M NaCI at pH 6.8. Fractions were tested and appropriately combined. The amino acid content was determined for those fractions containing protein so that the coupling efficiency for the various proteins at various coupling ratios could be determined. The results of the determinations are presented in Table 1.

**TABLE 1**

| Coupling Efficiencies of ITC-PGAFA with Various Proteins | | | |
|---|---|---|---|
| Protein | PGA Molar Excess | PGA Chain Number | Percent |
| Substitution | | | |
| Anti-CEA antibody | 1 | 0.77 | 77 |
| monoclonal 1.0 mg | 5 | 1.7 | 3 4 |
| | 10 | 3.1 | 31 |
| | 20 | 8.6 | 43 |
| | | | |
| Goat anti-rabbit antibody | | | |
| monoclonal 1.0 mg | 5 | 1.8 | 3 7 |
| | | | |
| Anti-β-hCG antibody | 10 | 4.6 | 46 |
| monoclonal 1.0 mg | 15 | 5.2 | 36 |
| monoclonal 10 mg | 15 | 7.8 | 52 |
| | | | |
| Anti-digoxin antibody | | | |
| monoclonal 1.0 mg | 15 | 8.1 | 54 |
| monoclonal 5.0 mg | 15 | 5.5 | 37 |
| | | | |
| Goat anti-mouse antibody | | | |
| polyclonal 1.0 mg | 15 | 4.3 | 29 |
| | | | |
| Anti-T4 antibody | | | |
| monoclonal 1.0 mg | 15 | 6.9 | 4 6 |
| | | | |
| Anti-T4 antibody | | | |
| polyclonal 7.0 mg | 15 | 13.8 | 9 2 |
| | | | |
| Rabbit Serum Albumin | | | |
| loaded with Theophylline | 15 | 7.8 | 5 2 |

Column 1 of Table 1 lists the quantity of protein used in the reactions to form the various capture reagents. Column 2 lists the mole excess of activated ITC-PGAFA that was reacted with the Column 1 protein. Column 3 provides the number of PGA chains attached per antibody by the reaction, calculated by amino acid analysis based upon a 40,000 average MW and 305 repeating glutamate residues. Column 4 provides a calculation of the percent efficiency of PGA chain substitution based upon the mole excess of activated PGA used in the reaction.

### Example 2

### Ion-capture Flow-Through Device for a Two-Step hCG Assay

### a. Preparation of the solid phase

Test sample application pads (glass fiber matrix) were treated with various concentrations of an aqueous solution of Merquat-100® polymeric ammonium compound. 100 mM Tris, 100 mM sodium chloride, 0.1% fish gelatin, 0.1% sucrose and 0.1% sodium azide. The application pads were allowed to dry, and the pads were overlaid upon a layer of absorbent material. Substantially the same procedure was used to prepare a flow-through solid phase device treated with Celquat® H-100 polymeric compound (comparative example). Alternative devices were prepared by treating the application pad with Merquat-100® polymeric quaternary ammonium compound (a cationic homopolymer of dimethyldiallylammonium chloride, 0.5% in water) immediately, before use.

### b. Preparation of the indicator reagent

The indicator reagent was a conjugate of goat anti-β-hCG antibody and alkaline phosphatase, diluted in 1% Brij®-35 polyoxyethylene (23) lauryl ether (Sigma), 100 mM Tris, 500 mM NaCI, 1 mM MgCl₂, 0.1 mM ZnCl₂, 0.1% NaN₃ and 0.5% non-fat dry milk at pH 7.2. The indicator reagent was filtered through a 0.22 µm filter before use.

In alternative indicator reagent preparations, dextran sulfate (MW 5,000) or heparin was included as a nonspecific binding blocker. The blocker was used to enhance the signal-to-noise ratio by inhibiting the binding of the labeled antibody to non-analyte.

### c. Preparation of the capture reagent

A monoclonal anti-β-hCG antibody-PGA capture reagent was prepared substantially in accordance with the method described in Example 1.c. above. Every five milliliters of the coupling reaction mixture was fractionated on a gel filtration chromatography column (2.4 x 54 cm, at a 0.4 ml/minute flow rate). The elution buffer contained 0.1 M sodium phosphate, 0.3 M NaCI and 0.05% NaN₃, at pH 8.5. The polymeric anion/antibody conjugate was diluted with 25 mM Tris, 100 mM NaCI, 1 mM MgCl₂, 0.1 mM ZnCl₂, 0.1% NaN₃, 10% normal mouse serum and 1% fish gelatin at pH 7.2. The capture reagent was filtered through a 0.22 µm filter before use.

### d. Immunoassay protocol

The capture reagent (80 µl) was mixed with an equal volume of test sample containing a known amount of hCG in normal human serum. The mixture was incubated at approximately 31-32°C for approximately twelve minutes. The specific binding reaction resulted in the formation of a capture reagent/analyte complex.

Each reaction mixture (80 µl) was then applied to a flow-through device, followed by a wash with Tris buffered saline (75 µl). The indicator reagent (50 µl) was then applied to the solid phase device and incubated for twelve minutes. The device was then washed two times.

An enzyme substrate (70 µl; 1.2 mM 4-methylumbelliferyl-phosphate in a solution of 100 mM AMP, 0.01% EDTA, 0.1% NaN₃, and 4.0 mM tetramisole at pH 10.3) was added, and the resulting rate of fluorescence was measured. The results of the assay are shown in Tables 2-4. The results demonstrated that as the hCG test sample concentration increased there was a corresponding increase in the formation of capture reagent/analyte/indicator reagent complex, and therefore, the amount of detectable label associated with the solid phase increased. The results show that the signal to noise ratio is improved by including a nonspecific binding blocker in the indicator reagent. Furthermore, the results demonstrated that the cationic homopolymer of dimethyldiallylammonium chloride was a preferred polymeric cation for the preparation of the solid phase for use in two-step assays wherein the device is subjected to one or more washings, e.g., the Merquat-100® polymeric ammonium compound has a nitrogen content of about 10% (exclusive of counter ion), whereas the Celquat® H-100 polymeric compound has a nitrogen content of about 1% (exclusive of counter ion).

**TABLE 2**

| hCG Ion-capture two-step Sandwich Assay Capture reagent: anti-β-hCG antibody-PGA (0.5 µg/test) Indicator reagent: alkaline phosphatase-labeled anti-β-hCG antibody (with and without nonspecific binding blocker) Solid phase: coated with a cationic homopolymer of dimethyldiallylammonium chloride immediately before use | | |
|---|---|---|
| | Rate of fluorescence (counts/sec/sec) | |
| hCG (mlU/ml) | 2% dextran sulfate | no blocker |
| 0 | 68 | 255 |
| 100 | 1028 | 1104 |

**TABLE 3**

| hCG Ion-capture two-step Sandwich Assay Capture reagent: anti-β-hCG antibody-PGA (0.5 µg/test) Indicator reagent: alkaline phosphatase-labeled anti-β-hCG antibody (with blocker) Solid phase: with varying cationic polymer concentration | | | | | |
|---|---|---|---|---|---|
| | Rate of fluorescence (counts/sec/sec) Merquat-100® polymeric ammonium compound (%w/v) | | | | |
| hCG (mlU/ml) | 0.02 | 0.04 | 0.2 | 0.4 | 0.6 |
| 0 | 34 | 31 | 26 | 30 | 39 |
| 100 | 514 | 578 | 627 | 661 | 647 |

**TABLE 4**

| hCG Ion-capture two-step Sandwich Assay Capture reagent: anti-β-hCG antibody-PGA Indicator reagent: alkaline phosphatase-labeled anti-β-hCG antibody Solid phase: with 0.125% Celquat® H-100 polymeric compound | | | |
|---|---|---|---|
| | Rate of fluorescence (counts/sec/sec) quantity of capture antibody (µg/test) | | |
| hCG (mlU/ml) | 0.268 | 0.402 | 0.652 |
| 0 | 86 | 100 | 115 |
| 100 | 186 | 202 | 259 |

### Example 3

### Ion-capture Flow-Through Device for Thyroia Stimulating Hormone (TSH) Assay

### a. Preparation of the solid phase

An application pad (glass fiber matrix) was treated with an aqueous solution of Merquat-100® polymeric ammonium compound substantially in accordance with the procedure described in Example 2.a. The pad was then overlaid upon a layer of absorbent material to complete the flow-through solid phase device.

### b. Preparation of the indicator reagent

The indicator reagent was a conjugate of goat anti-β-hCG antibody and alkaline phosphatase, diluted in 1% Brij®-35 polyoxyethylene (23) lauryl ether, 1% fish gelatin, 100 mM Tris, 500 mM NaCI, 1 mM MgCl₂, 0.1 mM ZnCl₂, 0.1% NaN₃ and 0.5% non-fat dry milk at pH 7.2. The indicator reagent was filtered through a 0.22 µm filter before use. Dextran sulfate (0.5%, MW 5,000) was added as a nonspecific binding blocker.

### c. Preparation of the capture reagent

The capture reagent was prepared by coupling a Protein A purified monoclonal anti-TSH antibody with carboxymethyl amylose (CMA; Polysciences, Inc., Warrington, PA). Coupling was performed using a water-soluble carbodiimide reagent (1-ethyl-3-(3-dimethylaminopropyl)carbodiimide; EDCI) substantially in accordance with the following procedure.

The coupling mixture contained an antibody solution (2 ml; 1 mg/ml in MES buffer [25 mM, 2-(N-Morpholino)ethanesulfonic acid) pH 5.5) and CMA (1.6 ml; 10 mg/ml in MES buffer). To the solution was added, with stirring, a freshly prepared EDCI solution (40 µl; 100 mg/ml in MES buffer). The reaction mixture was stirred at room temperature for 40 minutes. The reaction was quenched by adding a 25 % glycine solution (67 µl), and the product was then fractionated by gel filtration chromatography using a TSKgel G4000SW column (2.15 cm x 30 cm) fitted with a TSKguard column SW (2.15 cm x 7.5 cm; Anspec Co., Ann Arbor, Michigan). The column was eluted with PBS (0.1 M sodium phosphate, 0.3 M NaCI and 0.05% sodium azide, at pH 6.8). The purified Antibody/CMA capture reagent was diluted in a diluent containing 50 mM Tris, 300 mM NaCI, 1% bovine serum albumin, 2.5% fish gelatin and 0.1% NaN₃, at pH 7.5.

### d. Immunoassay protocol

The capture reagent (30 µl) and Tris buffered saline (100 Ml: 500 mM Tris, 300 mM NaCI and 0.1% NaH₃) were mixed with a test sample (50 µl) containing a known amount of hCG in normal human serum. The reaction mixture was incubated at approximately 33-34°C for approximately ten minutes. The specific binding reaction resulted in the formation of a capture reagent/analyte complex.

An aliquot of each reaction mixture (140 µl) was applied to a solid phase device, followed by a wash with Tris buffered saline (150 µl) The indicator reagent (70 µl) was applied to the device and incubated for approximately ten minutes. The device was then washed two times with buffer (100 µl each). The enzyme substrate (70 µl; 1.2 mM 4-methylumbelliferyl-phosphate in a solution of 100 mM AMP, 0.01% EDTA, 0.1% NaN₃, and 4.0 mM tetramisole at pH 10.3) was added, and the resulting rate of fluorescence was measured.

The results of the assay are shown in Table 5. The results demonstrated that as the concentration of TSH in the test sample increased, there was a corresponding increase in the formation of capture reagent/analyte/indicator reagent complex. Therefore, the amount of detectable label associated with the solid phase increased as the concentration of analyte increased. The results also demonstrated that the combination of Merquat-100® polymeric ammonium compound with polyacrylic acid or with carboxymethylamylose provided a solid phase and capture reagents which were advantageously used in two-step assays wherein the device is subjected to one or more washings or manipulations.

**TABLE 5**

| TSH Ion-capture Two-step Sandwich Assay (using polyacrylic acid or carboxymethylamylose polyanions) | | |
|---|---|---|
| | Rate of fluorescence (counts/sec/sec) | |
| TSH (mlU/ml) | carboxymethylamylose | polyacrylic acid |
| 0 | 7.1 | 6.4 |
| 0.5 | 13.3 | 12.1 |
| 2.0 | 34.7 | 28.7 |
| 10.0 | 147.5 | 119.8 |
| 40.0 | 513.9 | 442.6 |
| 100.0 | 1121.6 | 995.5 |

### e. TSH capturing efficiency

Radioiodinated TSH was used in the assay protocol, as described in Example 3. d, to demonstrate the more efficient TSH capturing of CMA-coupled antibodies than that of polyaspartic- and polyglutamic-coupled antibodies. The coupling of antibodies to the polyanions was performed substantially in accordance with the method described above (Example 3.c.) After the rate of fluorescence was measured at the end of the assay protocol, the radioactivity of TSH captured on the solid phase material was also measured by means of a scintillation spectrometer (Auto-Logic, Abbott Laboratories, North Chicago, IL). The results of this procedure are demonstrated in Table 5 (a).

**TABLE 5 (a)**

| Capture of Radiolabeled TSH in the Cationic Solid Phase Material | | |
|---|---|---|
| Polyanion-coupled anti-TSH antibody | %TSH captured | Rate of fluorescence (counts/sec/sec) |
| Carboxymethylamylose | 70 | 662 |
| Polyaspartic Acid | 1.5 | 37 |
| Polyglutamic Acid | 2.0 | 57 |

### Example 4

### Ion-capture Flow-Through Device for a One-Step hCG Assay

### a. Preparation of the solid phase

A glass fiber matrix was treated with an aqueous solution of Merquat-100® polymeric ammonium compound substantially in accordance with the procedure described in Example 2.a, above. The pad was then overlaid upon a layer of absorbent material to complete the device.

### b. Preparation of the indicator reagent

The indicator reagent was a goat anti-β-hCG antibody conjugated to alkaline phosphatase and diluted in 3.33% Brij®-35 polyoxyethylene (23) lauryl ether, 5 mM Tris, 1 mM MgCl₂, 0.1 mM ZnCl₂, 0.1% NaN₃ and 5% fish gelatin at pH 7.2. The indicator reagent was filtered through a 0.2 µm filter before use. In alternative indicator reagent preparations, carboxymethyl cellulose (MW 250.000) or carboxymethyl dextran was included as a nonspecific binding blocker.

### c. Preparation of the capture reagent

A monoclonal anti-hCG antibody-PGA capture reagent was prepared substantially in accordance with the method described in Example 3. c, above. The polymeric anion/antibody conjugate was diluted with 3.33% Brij®-35 polyoxyethylene (23) lauryl ether, 5 mM Tris, 500 mM NaCI, 1 mM MgCl₂, 0.1 mM ZnCl₂, 0.1% NaN₃, and 5% fish gelatin at pH 7.2. The enzyme substrate was 1.2 mM 4-methylumbelliferyl-phosphate in a solution of 100 mM AMP, 0.01% EDTA, 0.1% NaN₃, and 4.0 mM tetramisole at pH 10.3.

### d. Immunoassay protocol

The capture reagent (50 µl), indicator reagent (55 µl) and sample diluent buffer (35 µl; 75% normal calf serum, 25% normal goat serum and 0.2% NaN3, filtered through a 0.22 µm filter before use) were mixed with a test sample (30 µl) containing a known amount of hCG in normal human serum. The mixture was incubated at approximately 33-34°C for approximately fourteen minutes. The specific binding reaction resulted in the formation of a capture reagent/analyte/indicator reagent complex.

An aliquot of each reaction mixture (110 µl) was then applied to a solid phase device, followed by two washes with Tris buffered saline (75 µl). The enzyme substrate (65 µl) was added, and the resulting rate of fluorescence was measured.

The results of the assay are shown in Table 6. The results demonstrated that as the hCG test sample concentration increased there was a corresponding increase in the formation of capture reagent/analyte/indicator reagent complex, and therefore, the amount of detectable label associated with the solid phase increased. Furthermore, the results show that the signal to noise ratio was improved when a free polyanionic substance was included in the indicator reagent as a nonspecific binding blocker, even though the capture reagent was a polymeric anion/antibody conjugate.

**TABLE 6**

| hCG Ion-capture Sandwich Assay Capture reagent: anti-hCG antibody-PGA Indicator reagent: alkaline phosphatase-labeled anti-hCG antibody | | | | |
|---|---|---|---|---|
| | Rate of fluorescence (counts/sec/sec) % carboxymethyl cellulose in indicator reagent | | | |
| hCG (mlU/ml) | 0 | 0.01 | 0.25 | 0.5 |
| 0 | 37.2 | 23.8 | 17.2 | 13.3 |
| 10 | 76.8 | 58.4 | 48.8 | 42.1 |
| 1000 | 1803.6 | 1665.4 | 1692.2 | 1507.2 |

| | Rate of fluorescence (counts/sec/sec) % carboxymethyl dextran in indicator reagent | | | |
|---|---|---|---|---|
| hCG (mlU/ml) | 0 | 0.01 | 0.25 | 0.5 |
| 0 | 35.6 | 30.0 | 17.8 | 14.8 |
| 10 | 75.2 | 68.4 | 54.7 | 49.8 |
| 1000 | 1826.6 | 1851.2 | 1739.5 | 1646.6 |

### Example 5

### Ion-Capture Teststrip for an hCG Sandwich Assay

### a. Preparation of the solid phase

A rectangular zone on a central portion of a strip of nitrocellulose (5 µm pore size; Schleicher & Schuell; Dassel, Germany) was treated with an aqueous solution of 0.05% Merquat-100® polymeric ammonium compound and 10 mM Tris to form a positively charged capture or detection zone.

### b. Preparation of the indicator reagent

The indicator reagent was made of colloidal selenium particles coated with mouse monoclonal anti-hCG antibody. The indicator reagent was appropriately diluted (as determined by titer curve) in assay buffer containing 50 mM Tris, 2% lactose, 2% casein, 1% goal serum and 1% mouse serum at pH 8.4.

### c. Preparation of the capture reagent

A goat anti-β-hCG antibody was coupled to poly-L-glutamic acid using 1-ethyl-3-(3-dimethylaminopropyl)-carbodimide substantially in accordance with the method described in Example 3.c, above. The capture reagent was then appropriately diluted in the same diluent as the indicator reagent.

### d. Immunoassay protocol

The indicator reagent (50 µl) was mixed with an equal volume of capture reagent. The mixture was then combined with a series of samples (0, 50, 100 and 250 mlU/ml; 150 µl each) containing known amounts of hCG in normal human urine. The resultant reaction mixtures were incubated for five minutes at room temperature. The specific binding reaction resulted in the formation of a capture reagent/analyte/indicator reagent complex.

Each reaction mixture (250 µl) was then applied to one end of the prepared strip of nitrocellulose. The mixture was allowed to migrate through the strip to the capture zone and through the zone. Capture reagent and complexes thereof were retained at the capture zone, wherein the indicator reagent complexed with the retained capture reagent indicated the amount of analyte in the test sample as well as the the presence of analyte in the test sample. The 0 mlU/ml test sample produced no coloration of the capture zone. The 50, 100 and 250 mlU/ml test samples produced visible coloration of the capture zone.

### Example 6

### Ion-Capture Teststrip Device for a Phenylcyclidine (PCP) Assay

### a. Preparation of the solid phase

A rectangular zone on a central portion of an elongated strip of nitrocellulose (3 mm in width) was treated with an aqueous solution of 0.5% Merquat-100® polymeric ammonium compound to form a positively charged capture zone.

### b. Preparation of the indicator reagent

The indicator reagent was made of colloidal selenium particles coated with PCP antibody.

### c. Preparation of the capture reagent

A PCP antigen was conjugated to poly-glutamic acid substantially in accordance with the following method:

4,Hydroxy-Phenylcyclidine (1.1 mg; MW 259.37; 4.24 x 10⁻⁶ moles) was dissolved in tetrahydrofuran (THF; 0.5 ml). One-half milliliter of 10% phosgene in benzene was added (130 mole excess.) The reaction was allowed to proceed at room temperature for 2.5 hours. The solvent was evaporated under a stream of nitrogen to yield a residue of phenylcyclidine-4-chloroiormate.

The phenylcyclidine-4-chloroformate (1.1 mg) was dissolved in THF (0.5 ml). To this was added NH₂-PGAFA (1.7 mg; MW 11,798; 1.19 x 10⁻⁷ moles) dissolved in 1-methyl-2-pyrrolidinone (0.5 ml). The reaction was carried out overnight at room temperature and then rotavaporated to dryness. The product was dissolved in 0.1 M sodium phosphate (1.5 ml, pH 7.0). The precipitate was filtered, and the cloudy aqueous filtrate was extracted with methylene chloride until clear. The phenylcyclidine-PGA capture reagent, which was capable of binding with anti-phenylcyclidine antibody, was then diluted to 5 µg/ml in an assay buffer containing 25 mM Tris, 100 mM NaCI, 1 mM MgCl₂, 0.1 mM ZnCl₂, 0.1% NaN₃, and 1% fish gelatin at pH 7.2.

### d. Preparation of the assay device

An assay reagent pad (3 mm in width) or test sample application pad was prepared by soaking an absorbant material (Whatman PD075 glass fiber filter; Whatman Specialty Papers, Clifton, NJ) with the indicator reagent (2.5 mg/ml; 4% casein, 4% sucrose, 1% polyethylene glycol [MW 15,000-25,000] in 0.01 M Tris). The application pad was then air dried. The application pad and nitrocellulose where then assembled so that the reagent pad overlapped one end of the nitrocellulose by approximately one millimeter.

### e. Immunoassay protocol

The capture reagent (15 µl) and an equal volume of test sample, containing a known dilution of PCP in distilled water (1:10, 1:100, 1:1000, 1:10000), were mixed. The mixture was applied to the test sample application pad. The mixture was allowed to migrate through the pad and strip for at least ten five minutes. The competitive binding reaction resulted in the formation of capture reagent/indicator reagent complex and indicator reagent/analyte complex, wherein the amount of capture reagent/indicator reagent complex decreased as the amount of analyte in the test sample increased. The polyelectrolyte reaction resulted in the immobilization of the capture reagent/indicator reagent complex in the capture zone of the teststrip. Unbound indicator reagent and unreacted test sample components, as well as indicator reagent/analyte complex, passed through the capture zone. The assay results demonstrated that the higher the amount of PCP in the test sample, the lower the detectable signal at the capture zone. The assay results also demonstrated that a homogeneous specific binding reaction could take place in a solid phase teststrip device.

### Example 7

### Competitive Digoxin Assay Using Ion-Capture

### a. Preparation of the solid phase

Test sample application pads (glass fiber matrix) were overcoated with various concentrations of an aqueous solution of Merquat-100® polymeric ammonium compound, 100 mM Tris, 100 mM sodium chloride, 0.1% fish gelatin, 0.1% sucrose and 0.1% sodium azide. The application pads were allowed to dry and were then overlaid upon a layer of absorbent material to prepare flow-through devices.

### b. Preparation of the indicator reagent

The indicator reagent was a conjugate of digoxin dialdehyde and alkaline phosphatase, diluted in 50 mM Tris, 100 mM NaCI, 1 mM MgCl₂, 0.1 mM ZnCl₂, 0.1% NaN₃ and 0.1% bovine serum albumin at pH 7.5.

### c. Preparation of the capture reagent

The first capture reagent, a goat anti-digoxin antibody coupled to 1,4-phenylene diisothiocyanate activated poly-L-aspartic acid (ITC-PAA), was prepared substantially in accordance with the method described in Example 1.c. above. Poly-L-aspartic acid was used in place of poly-L-glutamic acid.

A second capture reagent was made of rabbit anti-goat IgG antibody coupled to poly-L-aspartic acid using EDCI substantially in accordance with the coupling protocol described in Example 3. c, above. An analyte-specific ancillary binding member (goat anti-digoxin antibody) was used together with this capture reagent to bind the analyte to the solid phase. In one embodiment, the capture reagent was a preformed complex of the negatively charged anti-goat antibody and the goat anti-digoxin antibody. Both the first and second capture reagents were appropriately diluted before use with 50 mM Tris, 50 mM NaCI, 0.1% NaN₃ and 0.1% bovine serum albumin at pH 7.5

### d. Immunoassay protocol

In assays using the first capture reagent, or direct capture system, the capture reagent (60 µl) was mixed with test sample (18 µl) containing a known amount of digoxin. The reaction mixture was incubated at approximately 33-34°C for about ten minutes. The specific binding reaction resulted in the formation of a capture reagent/analyte complex. The indicator reagent (60 µl) was then added to the reaction mixture, and the mixture was incubated for about another eleven minutes. The specific binding reaction resulted in the formation of capture reagent/indicator reagent complex in proportion to the amount of analyte present in the test sample. A portion of each reaction mixture (80 µl) was then applied to the solid phase, followed by two washes with Tris buffered saline (75 µl). An enzyme substrate (70 µl; 1.2 mM 4-methylumbelliferyl-phosphate in a solution of 100 mM AMP, 0.01% EDTA, 0.1% NaN₃, and 4.0 mM tetramisole at pH 10.3) was added, and the resulting rate of fluorescence was measured.

In an indirect assay using the second capture reagent, the preformed capture reagent/ancillary binding member complex (50 µl), indicator reagent (55 µl) and digoxin test sample (25 µl) were combined with sample diluent buffer (91 µl). The mixture was incubated for approximately nine minutes. The specific binding reaction resulted in the formation of capture reagent/ancillary binding member/analyte complex and capture reagent/ancillary binding member/indicator reagent complex in proportion to the amount of analyte present in the test sample. An aliquot of the reaction mixture (180 µl) was then applied to the solid phase, followed by two washes with Tris buffered saline (75 µl). The enzyme substrate (70 µl) was added, and the resulting rate of fluorescence was measured.

The results of the assay are shown in Table 7 The results demonstrated that as the digoxin test sample concentration increased there was a corresponding decrease in the formation of complex containing indicator reagent. Therefore, the amount of detectable label associated with the solid phase decreased with the increase of digoxin in the test sample.

**TABLE 7**

| Digoxin Ion-capture Competitive Assay | | |
|---|---|---|
| Protocol: | Semi-sequential One-Step | One-Step |
| Precoated Solid Phase: | 0.2% Merquat-100® | 0.2% Merquat-100® |
| Concentration of Antibody/Test: | 162 ng Goat anti-Digoxin | 90 ng Rabbit anti-Goat/ 64 ng Goat anti-Digoxin |
| Indicator Reagent: | Alkaline Phosphatase/Digoxin Conjugate | Alkaline Phosphatase/Digoxin Conjugate |

| | **Direct** | **Indirect** |
|---|---|---|
| Digoxin (ng/ml) | Rate of fluorescence (counts/sec/sec) | |
| 0 | 680 | 456 |
| 0.5 | 546 | 387 |
| 1 | 413 | 309 |
| 2 | 303 | 247 |
| 3 | 261 | 179 |
| 5 | 183 | 121 |

### Example 8

### Ion-capture Flow-Through Device for a Total T3 (Triiodothyronine) Competitive Assay

### a. Preparation of the solid phase

Test sample application pads (glass fiber matrix) were treated with various concentrations of an aqueous solution of Celquat® L-200 polymeric quaternary ammonium compound (comparative example) or Merquat-100® polymeric ammonium compound, 100 mM Tris, 100 mM sodium chloride; 0.1 % fish gelatin, 0.1% sucrose and 0.1% sodium azide. The application pads were allowed to dry, and the pads were overlaid upon a layer of absorbent material to form the individual assay devices.

### b. Preparation of the indicator reagent

The indicator reagent was a conjugate of T3 and alkaline phosphatase, diluted in 50 mM Tris, 100 mM NaCl, 1.0 mM MgCl₂, 0.1 mM ZnCl₂ and 1.0% bovine serum albumin at pH 7.5. Where indicated, dextran sulfate (MW 5,000) was included as a nonspecific binding blocker. The blocker was used to enhance the signal-to-noise ratio by inhibiting the binding of the labeled antibody to non-analyte.

### c. Preparation of the capture reagent

The capture reagent, an anti-T3 antibody coupled to polyaspartic acid (PAA-anti-T3 antibody), polyacrylic acid (PAcA-anti-T3 antibody) or carboxymethyl cellulose (CMA-anti-T3 antibody) anionic polymer molecules, was prepared substantially in accordance with the method described in the Example 3. c EDCI coupling method, with the exception that no chromatographic filtration of the capture reagent was performed. The capture reagent was diluted with 800 mM Tris, 50 mM NaCI, 0.1% NaN₃, 0.01% furosemide, 0.1% Tween®-20,1.0% bovine serum albumin and 0.08 mg/ml goat IgG at pH 7.4.

### d. Immunoassay protocol

The capture reagent (50 µl) was mixed with an equal volume of test sample, containing a known amount of Total T3, and sample diluent buffer (150 µl). The reaction mixture was incubated for approximately 15 minutes. The specific binding reaction resulted in the formation of a capture reagent/analyte complex.

Each reaction mixture (150 µl) was then applied to a solid phase. The indicator reagent (60 µl) was then applied to the solid phase and incubated for eight minutes. The device was then washed two times. An enzyme substrate (50 µl) was added, and the resulting rate of fluorescence was measured.

In an alternative assay format, the solid phase was also washed prior to the addition of the indicator reagent. In yet another assay format, the capture reagent and test sample were combined and incubated, followed by the addition of indicator reagent and further incubation prior to placing an aliquot of the reaction mixture on the solid phase.

The polyelectrolyte interaction of the capture reagent and the oppositely charged solid phase resulted in the immobilization of capture reagent and capture reagent complexes on the solid phase devices. An enzyme substrate (70 µl; 1.2 mM 4-methylumbelliferyl-phosphate in a solution of 100 mM AMP, 0.01% EDTA, 0.1% NaN₃, and 4.0 mM tetramisole at pH 10.3) was added, and the resulting rate of fluorescence was measured.

In each assay, the results demonstrated that as the Total T3 test sample concentration increased there was a corresponding increase in the formation of capture reagent/analyte complex, and therefore, the amount of detectable label associated with the solid phase decreased. Furthermore, the results show that the signal to noise ratio is improved by including a nonspecific binding blocker, dextran sulfate, in the indicator reagent.

**TABLE 9**

| Total T3 Competitive Assay Calibration data comparing one-step and two-step assay protocols | | |
|---|---|---|
| Protocol: | One-step | Two-step |
| Precoated Solid Phase: | 0.5% Celquat® | 0.2% Merquat-100® |
| Capture Antibody: (per test) | ITC-PGA anti-T3 antibody (0.25 µg) | EDAC-PAA anti-T3 antibody (0.02 µg) |
| Indicator Reagent: | no blocker | with 0.1% dextran sulfate |

| Calibrators concentration ng/ml total T3 | Rate of fluorescence (counts/sec/sec) | |
|---|---|---|
| 0 | 518 | 616 |
| 0.5 | 386 | 513 |
| 1.0 | 310 | 403 |
| 2.0 | 218 | 260 |
| 4.0 | 123 | 109 |
| 8.0 | 71 | 48 |

**TABLE 10**

| Total T3 Competitive Two-Step Assay comparison of indicator reagents with and without a nonspecific binding blocker | | |
|---|---|---|
| Precoated Solid Phase: | 0.2% Merquat-100® | 0.2% Merquat-100® |
| Capture Antibody: (per test) | EDAC-PAA anti-T3 antibody (0.02 µg) | EDAC-PAA anti-T3 antibody (0.02 µg) |
| Indicator Reagent: T3 alkaline phosphatase | no blocker | with 0.1% dextran sulfate |
| dilution: | 1:400 | 1:150 |

| Calibrators concentration ng/ml total T3 | Rate of fluorescence (counts/sec/sec) | |
|---|---|---|
| 0 | 641 | 536 |
| 2.0 | 361 | 220 |
| 8.0 | 81 | 39 |

**TABLE 11**

| Total T3 Competitive Assay Calibration data comparing different T3 capture reagents | | | |
|---|---|---|---|
| Capture Antibody: (per test) | PAA-anti-T3 antibody (0.013 µg) | PAcA-anti-T3 antibody (0.015 µg) | CMA-anti-T3 antibody (0.013 µg) |

| Calibrators concentration ng/ml total T3 | Rate of fluorescence (counts/sec/sec) | | |
|---|---|---|---|
| 0 | 509 | 544 | 507 |
| 0.5 | 401 | 443 | 394 |
| 1.0 | 332 | 344 | 322 |
| 2.0 | 203 | 219 | 204 |
| 4.0 | 94 | 107 | 99 |
| 8.0 | 47 | 57 | 51 |

## Claims

1. A method for determining the presence or amount of an analyte in a test sample, comprising the steps of:
a) providing
(i) a capture reagent, comprising a first binding member conjugated to a polymeric anion substance,
(ii) an indicator reagent, comprising a second binding member and a detectable label, and
(iii) a solid phase material containing a reaction site comprising a polymeric cation substance having a nitrogen content of at least about ten percent;
wherein said first and second specific binding members are selected from binding members capable of forming a complex with the analyte in a sandwich assay, a competitive assay or an indirect assay, and thereby forming a detectable complex in proportion to the presence or amount of the analyte in the test sample;
b) contacting said solid phase with said capture reagent and the test sample, whereby said polymeric cation of said solid phase attracts and attaches to said polymeric anion of said capture reagent, thereby immobilizing said capture reagent and complexes thereof;
c) contacting said solid phase with said indicator reagent, whereby said indicator reagent becomes bound to said immobilized capture reagent or complex thereof in proportion to the amount of analyte present in the test sample; and
d) detecting said indicator reagent associated with said solid phase or unbound indicator reagent to determine the presence or amount of the analyte in the test sample.

2. The method according to Claim 1, wherein said polymeric cation substance is a polymeric quaternary ammonium compound.

3. The method according to Claim 1, wherein said polymeric cation substance is incorporated in or on said solid phase immediately before performance of the assay process.

4. The method according to Claim 1, further comprising the step of combining the test sample and said capture reagent to form a reaction mixture, wherein said reaction mixture is contacted to said solid phase.

5. The method according to Claim 1, further comprising the addition of an ancillary specific binding member, wherein said ancillary specific binding member is capable of binding the analyte and is capable of binding a member selected from the group consisting of said indicator reagent or said capture reagent in an indirect assay.

6. A method for determining the presence or amount of an analyte in a test sample, comprising the steps of:
a) providing
(i) a capture reagent, comprising a first binding member conjugated to a polymeric anion substance,
(ii) an indicator reagent, comprising a second binding member and a detectable label,
(iii) a non-specific binding blocker comprising an unbound polymeric anion material, and
(iv) a solid phase material containing a reaction site comprising a polymeric cation substance having a nitrogen content of at least about two percent;
wherein said non-specific binding blocker inhibits the non-specific binding of reagents to said solid phase, and wherein said first and second specific binding members are selected from binding members capable of forming a complex with the analyte in a sandwich assay, a competitive assay or an indirect assay, and thereby forming a detectable complex in proportion to the presence or amount of the analyte in the test sample;
b) contacting said solid phase with said capture reagent and the test sample, whereby said polymeric cation of said solid phase attracts and attaches to said polymeric anion of said capture reagent, thereby immobilizing said capture reagent and complexes thereof;
c) washing said solid phase;
d) contacting said solid phase with said indicator reagent, whereby said indicator reagent becomes bound to said immobilized capture reagent or complex thereof in proportion to the amount of analyte present in the test sample; and
e) detecting said indicator reagent associated with said solid phase or unbound indicator reagent to determine the presence or amount of the analyte in the test sample.

7. The method according to Claim 6, further comprising the step of combining the test sample and said capture reagent to form a reaction mixture, wherein said reaction mixture is contacted to said solid phase.

8. The method according to Claim 6, further comprising the addition of an ancillary specific binding member, wherein said ancillary specific binding member is capable of binding the analyte and is capable of binding a member selected from the group consisting of said indicator reagent or said capture reagent in an indirect assay.

## Patentansprüche

1. Verfahren zur Bestimmung der Anwesenheit oder Menge eines Analyten in einer Testprobe, das folgende Schritte umfaßt:
a.) Bereitstellen
(i) eines Einfangreagenzes, das ein erstes bindendes Glied umfaßt, das an eine polymere anionische Substanz konjugiert ist,
(ii) eines Indikatorreagenzes, das ein zweites bindendes Glied und eine nachweisbare Markierung umfaßt, und
(iii) eines Festphasenmaterials, das eine Reaktionsstelle enthält, die eine polymere kationische Substanz mit einem Stickstoffgehalt von wenigstens ungefähr zehn Prozent umfaßt;
worin das erste und das zweite spezifisch bindende Glied aus Bindungsgliedern gewählt sind, die zur Ausbildung eines Komplexes mit dem Analyten in einem Sandwich-Assay, einem Kompetitiv-Assay oder in einem indirekten Assay befähigt sind, und die hierdurch einen nachweisbaren Komplex proportional zur Anwesenheit oder Menge des Analyten in der Testprobe ausbilden;
b) In-Kontakt-Bringen der festen Phase mit dem Einfangreagenz und der Testprobe, wodurch das polymere Kation der festen Phase das polymere Anion des Einfangreagenzes anzieht und sich an dieses bindet, wodurch das Einfangreagenz und dessen Komplexe immobilisiert werden;
c) In-Kontakt-Bringen der festen Phase mit dem Indikatorreagenz, wodurch das Indikatorreagenz an das immobilisierte Einfangreagenz oder dessen Komplex proportional zur Menge an in der Testprobe vorhandenem Analyt gebunden wird; und
d) Nachweis von mit der festen Phase assoziiertem Indikatorreagenz oder von ungebundenem Indikatorreagenz, um die Anwesenheit oder Menge von Analyt in der Testprobe zu bestimmen.

2. Verfahren nach Anspruch 1, worin die polymere kationische Substanz eine polymere quaternäre Ammoniumverbindung ist.

3. Verfahren nach Anspruch 1, worin die polymere kationische Substanz in oder auf der festen Phase unmittelbar vor der Durchführung des Assayverfahrens eingebaut wird.

4. Verfahren nach Anspruch 1, das des weiteren den Schritt des Zusammengebens der Testprobe und des Einfangreagenzes unter Ausbildung einer Reaktionsmischung umfaßt, wobei die Reaktionsmischung mit der festen Phase in Kontakt gebracht wird.

5. Verfahren nach Anspruch 1, das des weiteren die Zugabe eines spezifisch bindenden Hilfsgliedes umfaßt, wobei das spezifisch bindende Hilfsglied zur Bindung des Analyten fähig ist und zur Bindung eines Gliedes fähig ist, das aus der Gruppe gewählt ist, die aus dem Indikatorreagenz oder dem Einfangreagenz in einem indirekten Assay besteht.

6. Verfahren zur Bestimmung der Anwesenheit oder Menge eines Analyten in einer Testprobe, das folgende Schritte umfaßt:
a) Bereitstellen
(i) eines Einfangreagenzes, das ein erstes bindendes Glied umfaßt, das an eine polymere anionische Substanz konjugiert ist,
(ii) eines Indikatorreagenzes, das ein zweites bindendes Glied und eine nachweisbare Markierung umfaßt,
(iii) eines nicht-spezifischen Bindungsblockers, der ein ungebundenes polymeres anionisches Material umfaßt, und
(iv) eines Festphasenmaterials, das eine Reaktionsstelle enthält, die eine polymere kationische Substanz umfaßt, die einen Stickstoffgehalt von wenigstens ungefähr zwei Prozent aufweist;
wobei der nicht-spezifische Bindungsblocker die nicht-spezifische Bindung von Reagenzien an die feste Phase hemmt, und wobei das erste und das zweite spezifisch bindende Glied aus Bindungsgliedern gewählt sind, die zur Ausbildung eines Komplexes mit dem Analyten in einem Sandwich-Assay, einem Kompetitiv-Assay oder in einem indirekten Assay befähigt sind, und hierdurch Ausbildung eines nachweisbaren Komplexes proportional zur Anwesenheit oder Menge des Analyten in der Testprobe;
b) In-Kontakt-Bringen der festen Phase mit dem Einfangreagenz und der Testprobe, wodurch das polymere Kation der Festphase das polymere Anion des Einfangreagenzes anzieht und sich an dieses bindet, wodurch das Einfangreagenz und dessen Komplexe immobilisiert werden;
c) Waschen der Festphase;
d) In-Kontakt-Bringen der Festphase mit dem Indikatorreagenz, wodurch das Indikatorreagenz an das immobilisierte Einfangreagenz oder dessen Komplex proportional zur in der Testprobe vorhandenen Analytmenge gebunden wird; und
e) Nachweis des Indikatorreagenzes, das mit der festen Phase oder dem ungebundenen Indikatorreagenz assoziiert ist, um die Anwesenheit oder Menge an Analyt in der Testprobe zu bestimmen.

7. Verfahren nach Anspruch 6, das des weiteren den Schritt der Kombination der Testprobe und des Einfangreagenzes unter Ausbildung einer Reaktionsmischung umfaßt, wobei die Reaktionsmischung mit der festen Phase in Kontakt gebracht wird.

8. Verfahren nach Anspruch 6, das des weiteren die Zugabe eines spezifisch bindenden Hilfsgliedes umfaßt, wobei das spezifisch bindende Hilfsglied zur Bindung des Analyten und zur Bindung eines Gliedes befähigt ist, das aus der Gruppe gewählt ist, die aus dem Indikatorreagenz oder dem Einfangreagenz in einem indirekten Assay besteht.

## Revendications

1. Procédé pour déterminer la présence ou la quantité d'un composé à analyser dans un échantillon à tester, comprenant les étapes de :
a) fournir
(i) un réactif de capture, comprenant un premier élément de liaison conjugué à une substance anionique polymère,
(ii) un réactif indicateur, comprenant un second élément de liaison et un marqueur détectable, et
(iii) une matière en phase solide contenant un site réactif comprenant une substance cationique polymère ayant une teneur en azote d'au moins environ dix pour-cent ;
caractérisé en ce que lesdits premier et second éléments de liaison spécifiques sont choisis parmi des éléments de liaison capables de former un complexe avec, le composé à analyser dans une analyse sandwich, une analyse compétitive ou une analyse indirecte, et en formant ainsi un complexe détectable en proportion de la présence ou de la quantité du composé à analyser dans un échantillon à tester ;
b) mettre en contact ladite phase solide avec ledit réactif de capture et l'échantillon à tester, ledit cation polymère de ladite phase solide attirant et s'attachant audit anion polymère dudit réactif de capture, en immobilisant ainsi ledit réactif de capture et ses complexes ;
c) mettre en contact ladite phase solide avec ledit réactif indicateur, ledit réactif indicateur devenant ainsi lié audit réactif de capture immobilisé ou à son complexe en proportion de la quantité de composé à analyser présent dans l'échantillon à tester ; et
d) détecter ledit réactif indicateur associé avec ladite phase solide ou le réactif indicateur non lié pour déterminer la présence ou la quantité de composé à analyser dans l'échantillon à tester.

2. Procédé selon la revendication 1, caractérisé en ce que ladite substance cationique polymère est un composé ammonium quaternaire polymère.

3. Procédé selon la revendication 1, caractérisé en ce que ladite substance cationique polymère est introduite dans ou sur ladite phase solide immédiatement avant la réalisation du procédé d'analyse.

4. Procédé selon la revendication 1, comprenant en outre l'étape de combiner l'échantillon à tester et ledit réactif de capture pour former un mélange réactionnel, caractérisé en ce que ledit mélange réactionnel est mis en contact avec ladite phase solide.

5. Procédé selon la revendication 1, comprenant l'addition d'un élément de liaison spécifique auxiliaire, caractérisé en ce que ledit élément de liaison spécifique auxiliaire est capable de lier le composé à analyser et est capable de lier un élément choisi dans le groupe formé par ledit réactif indicateur et ledit réactif de capture dans une analyse indirecte.

6. Procédé pour déterminer la présence ou la quantité d'un composé à analyser dans un échantillon à tester, comprenant les étapes de :
a) fournir
(i) un réactif de capture, comprenant un premier élément de liaison conjugué à une substance anionique polymère,
(ii) un réactif indicateur, comprenant un second élément de liaison et un marqueur détectable,
(iii) un agent de blocage de liaison non spécifique comprenant un composé anionique polymère non lié,
(iv) une matière en phase solide comprenant un site de comprenant une substance cationique polymère ayant une teneur en azote d'au moins environ deux pour-cent ;
caractérisé en ce que ledit agent de blocage de liaison non spécifique inhibe la liaison non spécifique des réactifs à ladite phase solide, et lesdits premier et second éléments de liaison particuliers sont choisis parmi des éléments de liaison capables de former un complexe avec le composé à analyser dans une analyse sandwich, une analyse compétitive ou une analyse indirecte, et en formant ainsi un complexe détectable en proportion de la présence ou de la quantité du composé à analyser dans un échantillon à tester ;
b) mettre en contact ladite phase solide avec ledit réactif de capture et l'échantillon à tester, ledit cation polymère de ladite phase solide attirant et s'attachant audit anion polymère dudit réactif de capture, en immobilisant ainsi ledit réactif de capture et ses complexes ;
c) laver ladite phase solide ;
d) mettre en contact ladite phase solide avec ledit réactif indicateur, ledit réactif indicateur devenant ainsi lié audit réactif de capture immobilisé ou à son complexe en proportion de la quantité de composé à analyser présent dans l'échantillon à tester ; et
e) détecter ledit réactif indicateur associé avec ladite phase solide ou le réactif indicateur non lié pour déterminer la présence ou la quantité de composé à analyser dans l'échantillon à tester.

7. Procédé selon la revendication 6, comprenant en outre l'étape de combiner l'échantillon à tester et ledit réactif de capture pour former un mélange réactionnel, caractérisé en ce que ledit mélange réactionnel est mis en contact avec ladite phase solide.

8. Procédé selon la revendication 6, comprenant l'addition d'un élément de liaison spécifique auxiliaire, caractérisé en ce que ledit élément de liaison spécifique auxiliaire est capable de lier le composé à analyser et est capable de lier un élément choisi dans le groupe formé par ledit réactif indicateur et ledit réactif de capture dans une analyse indirecte.
